# EUROPEAN PATENT SPECIFICATION

(11) **EP 4 032 522 B1**
(45) Date of publication and mention of the grant of the patent: **29.04.2026**
(21) Application number: 20865424.4
(22) Date of filing: 18.09.2020
(51) Int. Cl.: A61K 6/884, A61K 6/889, A61K 6/887

(54) **CURABLE COMPOSITION FOR DENTISTRY**
HÄRTBARE ZUSAMMENSETZUNG FÜR DIE ZAHNMEDIZIN
COMPOSITION DURCISSABLE POUR DENTISTERIE

(30) Priority: 19.09.2019 JP 2019170592
(43) Date of publication of application: 27.07.2022
(73) Proprietor: Kuraray Noritake Dental Inc., Kurashiki-shi, Okayama 710-0801 (JP)
(72) Inventor: KAJIKAWA, Tatsuya, Tainai-shi, Niigata 959-2653 (JP); HORIGUCHI, Hirotaka, Tainai-shi, Niigata 959-2653 (JP)
(74) Representative: D Young & Co LLP
(86) International application number: PCT/JP2020/035544
(87) International publication number: WO 2021/054457

(56) References cited:
- EP-A1- 3 603 607
- WO-A1-2016/163540
- WO-A1-2018/105463
- WO-A1-2018/181710
- JP-A- 2002 097 109
- JP-A- 2014 114 259
- US-A1- 2002 061 937
- US-A1- 2013 225 699

## Description

### TECHNICAL FIELD

The present invention relates to a dental curable composition that can be suitably used in dental applications such as dental materials, particularly dental composite resins, for partial or whole replacement of natural tooth.

### BACKGROUND ART

A dental composite resin, a mixture of primarily polymerizable monomer, filler, and polymerization initiator, is a dental material that has become the material of choice for restoration of missing tooth parts and cavities. The following are some of the improvements desired for dental composite resins. Specifically, a dental composite resin, in the form of a cured article after polymerization and cure, is desired to have improved mechanical strength, reduction of polymerization shrinkage upon cure, reduced water absorption and staining, and reduced wear on opposing teeth. Before polymerization and cure, a dental composite resin itself is desired to have improved ease of handling (for example, the properties allowing clinicians and denturists to easily handle the resin without the resin adhering to dental instruments or being too sticky while retaining the property to easily fill cavities).

There is also a strong desire to reduce polymerization shrinkage as much as possible because polymerization shrinkage causes a contraction gap that occurs when a dental composite resin pulls away from the surface it is adhering to. Formation of a contraction gap leads to various problems, including secondary caries, stimulation of tooth pulp, staining, and loose restorations.

Techniques are available that reduce such polymerization shrinkage. For example, Patent Literature 1 describes a self-adhesive dental composite resin that uses a specific long-chain polyfunctional polymerizable monomer to reduce polymerization shrinkage stress while having desirable adhesive properties to tooth structure and providing desirable mechanical strength to the cured product. Patent Literature 2 and 3 descibe dental curable compostions.

### CITATION LIST

### Patent Literature

Patent Literature 1: JP 2012-171885 A
Patent Literature 2: US 2002/061937 A1
Patent Literature 3: WO 2018/181710 A1

### SUMMARY OF INVENTION

### Technical Problem

However, studies by the present inventors revealed that the current techniques still have areas for improvement. Specifically, while a dental curable composition using a long-chain polyfunctional polymerizable monomer such as that described in Patent Literature 1 can have a reduced polymerization shrinkage stress by increasing the content of the long-chain polyfunctional polymerizable monomer, a cured product of the dental curable composition has low mechanical strength, and the water absorbency of the cured product increases and makes the cured product less durable in oral cavity.

It is accordingly an object of the present invention to provide a dental curable composition, suited for applications such as dental composite resins, having a small polymerization shrinkage stress and a consistency to easily fill cavities, and that, in the form of a cured product, has excellent mechanical strength with low water absorbency while causing reduced wear on opposing teeth.

### Solution to Problem

The present inventors conducted intensive studies to achieve the foregoing object, and found that a dental composite resin containing two different types of specific polymerizable monomers can provide a dental composite resin having a small polymerization shrinkage stress and a consistency to easily fill cavities, and that, in the form of a cured product, has excellent mechanical strength with low water absorbency while causing reduced wear on opposing teeth. The present invention was completed after further studies based on this finding.

Specifically, the present invention is defined in the appended claims.

### Advantageous Effects of Invention

The present invention can provide a dental curable composition, suited for applications such as dental composite resins, having a small polymerization shrinkage stress and a consistency to easily fill cavities, and that, in the form of a cured product, has excellent mechanical strength with low water absorbency while causing reduced wear on opposing teeth.

### DESCRIPTION OF EMBODIMENTS

The invention is defined by the independent claim. The dependent claims describe preferred embodiments. The following describes the present invention in detail. A dental curable composition of the present invention is a dental curable composition comprising a polymerizable monomer (A), a filler (B), and a polymerization initiator (C),
the polymerizable monomer (A) containing a polyfunctional (meth)acrylic monomer having no aromatic ring but having a moiety (X) constituted by an alkoxylene repeating unit having 1 to 15 carbon atoms (hereinafter, also referred to simply as "polyfunctional (meth)acrylic monomer (A-1)"), and a polyfunctional (meth)acrylic monomer (A-2) having an aromatic ring but no hydroxyl group (hereinafter, also referred to simply as "polyfunctional (meth)acrylic monomer (A-2)"),
the moiety (X) having a C/O of 2.5 to 10 as a ratio of a total number of carbon atoms and a total number of oxygen atoms in the moiety (X) (a ratio of a total number of carbon atoms to a total number of oxygen atoms; "C/O" in this specification),
the moiety (X) having a backbone with a total of 25 to 60 constituent carbon atoms and oxygen atoms altogether (C + O),
the polyfunctional (meth)acrylic monomer (A-1) being 3 to 60 parts by mass in 100 parts by mass of the polymerizable monomer (A).

As used herein, "(meth)acryl" is intended to be inclusive of both methacryl and acryl. As used herein, "(meth)acrylic monomer " is intended to be inclusive of both (meth)acrylic acid ester and derivatives of (meth)acrylamide.

With this configuration, a dental curable composition suited for applications such as dental composite resins can be provided that has a small polymerization shrinkage stress and a consistency to easily fill cavities, and that, in the form of a cured product, has excellent mechanical strength with low water absorbency while causing reduced wear on opposing teeth. As used herein, "consistency" describes the extent of spread of a paste of when a paste of a dental curable composition is squashed under a certain load, and higher consistency values mean that a paste of a dental curable composition is softer, and lower consistency values mean that a paste of a dental curable composition is harder.

A possible explanation for these desirable effects is as follows, though this is not to be construed as limiting the present invention in any ways. Specifically, an increase in the flexural modulus of a cured product of a dental curable composition can be reduced when the curable composition contains a specific amount of polyfunctional (meth)acrylic monomer (A-1). Apparently, this makes it possible to effectively reduce polymerization shrinkage stress, and, because the cured product of the dental curable composition does not become overly hydrophilic, water absorbency can be reduced. Another component is that a dental curable composition can usually produce a cured product having high mechanical strength when the curable composition uses the polyfunctional (meth)acrylic monomer (A-2) having an aromatic ring but no hydroxyl group, and that the polyfunctional (meth)acrylic monomer (A-2) has a limited interaction with the polyfunctional (meth)acrylic monomer (A-1) in monomer form. A paste of the dental curable composition using these two different monomers has a low viscosity, and imparts less stickiness. This should make it easier to fill cavities, and provide a dental curable composition suited for applications such as dental composite resins. A cured product of a dental curable composition of the present invention has a limited increase of flexural modulus, and can reduce wear of opposing teeth. A dental curable composition of the present invention has desirable curability, and can reduce wear of opposing teeth by preventing the filler from separating from the cured product, and acting as an abrasive and wearing the opposing teeth.

### Polymerizable Monomer (A)

The polymerizable monomer (A) in a dental curable composition of the present invention contains a polyfunctional (meth)acrylic monomer (A-1) having no aromatic ring but having a moiety (X) constituted by an alkoxylene repeating unit having 1 to 15 carbon atoms, and a polyfunctional (meth)acrylic monomer (A-2) having an aromatic ring but no hydroxyl group.

### Polyfunctional (Meth)acrylic Monomer (A-1)

The polyfunctional (meth)acrylic monomer (A-1) in the present invention has no aromatic ring but has a moiety (X) constituted by an alkoxylene repeating unit having 1 to 15 carbon atoms. The moiety (X) has a C/O of 2.5 to 10 as a ratio of a total number of carbon atoms and a total number of oxygen atoms in the moiety (X). The moiety (X) has a backbone with a total of 25 to 60 constituent carbon atoms and oxygen atoms altogether.

The alkoxylene repeating unit of the moiety (X) has 1 to 15 carbon atoms, preferably 2 to 10 carbon atoms, more preferably 2 to 6 carbon atoms, even more preferably 2 to 5 carbon atoms, particularly preferably 3 to 5 carbon atoms. With the number of carbon atoms having these upper limits, a cured product of the dental curable composition can have a reduced flexural modulus, and a reduced polymerization shrinkage stress upon cure. With the foregoing lower limits of the number of carbon atoms, a monomer having high storage stability can be obtained.

The moiety (X) may be linear, branched, or cyclic. However, the moiety (X) is preferably linear. The moiety (X) may have one or more substituents, or may be unsubstituted. When the moiety (X) has a substituent, the number of substituents is not particularly limited, and may be 1 to 8, 1 to 6, or 1 to 3. Examples of substituents include halogen atoms, an alkyl group having 1 to 6 carbon atoms, a C1 to C6 alkyl group substituted with a halogen atom, and an alkenyl group having 2 to 6 carbon atoms.

The value of C/O as a ratio of a total number of carbon atoms and a total number of oxygen atoms in the moiety (X) is 2.5 to 10, preferably 2.5 to 8, more preferably 3 to 6, even more preferably 3 to 5. With C/O having these upper limits, a dental curable composition having high mechanical strength can be obtained. With the foregoing lower limits of C/O, the dental curable composition can produce a cured product having a low water absorbency, and an increase of flexural modulus in the cured product can be inhibited. This makes it possible to effectively reduce polymerization shrinkage stress, and the dental curable composition causes reduced wear on opposing teeth while providing high mechanical strength.

The moiety (X) has a backbone with a total of 25 to 60, preferably 27 to 59, more preferably 30 to 55, even more preferably 35 to 53, particularly preferably 40 to 50 constituent carbon atoms and oxygen atoms altogether. By setting these upper limits for the total number of constituent carbon atoms and oxygen atoms in the backbone, the polyfunctional (meth)acrylic monomer (A-1) can have a reduced viscosity, and a paste of the dental curable composition can have a low viscosity with less stickiness. By setting the foregoing lower limits for the total number of constituent carbon atoms and oxygen atoms in the backbone, the dental curable composition can produce a cured product that has a low flexural modulus, and causes less wear on opposing teeth, and the dental curable composition can have a reduced polymerization shrinkage stress upon cure.

It is particularly preferable that the polyfunctional (meth)acrylic monomer (A-1) be a compound represented by the following general formula (1) because of advantages such as availability, reduction of polymerization shrinkage stress, superior mechanical strength of a cured product, lower water absorbency, even less wear on opposing teeth, and the consistency to easily fill cavities. 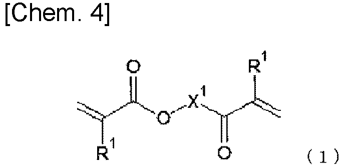
wherein R¹ represents a hydrogen atom or a methyl group,
X¹ is constituted by an alkoxylene repeating unit having 1 to 15 carbon atoms, in which C/O as a ratio of a total number of constituent carbon atoms and a total number of constituent oxygen atoms is 2.5 to 10, and X¹ has a backbone with a total of 25 to 60 constituent carbon atoms and oxygen atoms altogether, and
a plurality of R¹ may be the same or different from each other.

In general formula (1), X¹ is constituted by an alkoxylene repeating unit. For the same reasons discussed above for the alkoxylene repeating unit of the moiety (X), the alkoxylene repeating unit has 1 to 15 carbon atoms, preferably 2 to 10 carbon atoms, more preferably 2 to 6 carbon atoms, even more preferably 2 to 5 carbon atoms, particularly preferably 3 to 5 carbon atoms.

X¹ may be a divalent polyether group constituted by the same alkoxylene repeating unit, or may be a divalent random, block, or alternating copolyether group constituted by different alkoxylene repeating units. Examples of the former include a divalent polyether group represented by -[CH₂CH₂CH₂CH₂O]ₛ- (s = 5 to 11). Examples of the latter include a divalent polyether group represented by -[CH₂O]ₜ-[CH₂CH₂CH₂O]ᵤ-(t = 1 to 5, u = 1 to 11; the sum of t and u must satisfy the condition that the polyether group has a total of 25 to 59 carbon atoms and oxygen atoms altogether).

The alkoxylene repeating unit includes preferably an alkoxylene repeating unit represented by -CH₂O-, -CH₂CH₂O-, -CH₂CH(CH₃)O-, -CH₂CH₂CH₂O-, - CH₂CH₂CH₂CH₂O-, -CH₂CH(R⁴)CH(R⁵)O-, or -CH₂CH(R⁴)CH(R⁵)CH₂O-. Here, R⁴ is a hydrogen atom when R⁵ is a methyl group, and is a methyl group when R⁵ is a hydrogen atom. For advantages such as availability and enhancement of the effectiveness of the present invention, preferred are -CH₂CH₂CH₂O-, -CH₂CH₂CH₂CH₂O-, - CH₂CH(R⁴)CH(R⁵)O-, and -CH₂CH(R⁴)CH(R⁵)CH₂O-, and more preferred are - CH₂CH₂CH₂CH₂O- and -CH₂CH(R⁴)CH(R⁵)CH₂O-.

In general formula (1), X¹ may be linear, branched, or cyclic. However, X¹ is preferably linear. X¹ may have one or more substituents, or may be unsubstituted.

For the same reasons discussed above for the ratio C/O of a total number of carbon atoms and a total number of oxygen atoms in moiety (X), the ratio C/O of a total number of carbon atoms and a total number of oxygen atoms in X¹ of general formula (1) is 2.5 to 10, preferably 2.5 to 8, more preferably 3 to 6, even more preferably 3 to 5.

For the same reasons discussed above for the total number of constituent carbon atoms and oxygen atoms in the backbone of moiety (X), the total number of constituent carbon atoms and oxygen atoms in the backbone of X¹ in general formula (1) is 25 to 60, preferably 27 to 59, more preferably 30 to 55, even more preferably 35 to 53, particularly preferably 40 to 50.

It is preferable that the polyfunctional (meth)acrylic monomer (A-1) be a polybutylene glycol dimethacrylate represented by the following general formula (4) (average number of moles of butylene glycol group added: 5 to 12) because of advantages such as availability, reduction of polymerization shrinkage stress, superior mechanical strength of a cured product, lower water absorbency, even less wear on opposing teeth, and the consistency to easily fill cavities.

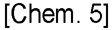

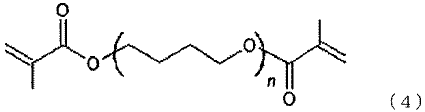

The average number of moles of butylene glycol group added (n) in the polybutylene glycol dimethacrylate is more preferably 5 to 11, even more preferably 7 to 11. The polyfunctional (meth)acrylic monomer (A-1) does not have an aromatic ring. Here, the aromatic ring may refer to the same aromatic ring contained in the polyfunctional (meth)acrylic monomer (A-2) having an aromatic ring but no hydroxyl group (described later).

The content of polyfunctional (meth)acrylic monomer (A-1) per 100 parts by mass of polymerizable monomer (A) is 3 to 60 parts by mass, preferably 5 to 60 parts by mass, more preferably 10 to 60 parts by mass, even more preferably 20 to 60 parts by mass, particularly preferably 30 to 50 parts by mass. By setting these lower limits, the polymerization shrinkage stress of the dental curable composition can be effectively reduced. By setting the foregoing upper limits, the dental curable composition can have less stickiness, and the cured product can have a sufficiently high mechanical strength. The polyfunctional (meth)acrylic monomer (A-1) may be used alone, or two or more thereof may be used in combination.

### Polyfunctional (Meth)acrylic Monomer (A-2)

The polyfunctional (meth)acrylic monomer (A-2) in the present invention has an aromatic ring but no hydroxyl group. Examples of the aromatic ring include a benzene ring, and aromatic rings containing fused rings, such as those of naphthalene.

Examples of the polyfunctional (meth)acrylic monomer (A-2) having an aromatic ring but no hydroxyl group include 2,2-bis((meth)acryloyloxyphenyl)propane, 2,2-bis(4-(meth)acryloyloxypolyethoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, 2,2-bis(4-(meth)acryloyloxydipropoxyphenyl)propane, 2-(4-(meth)acryloyloxydipropoxyphenyl)-2-(4-(meth)acryloyloxytriethoxyphenyl)propane, 9,9-bis[4-(2-(meth)acryloyloxyethoxy)phenyl]fluorene, 9,9-bis[4-(2-(meth)acryloyloxypolyethoxy)phenyl]fluorene, diphenylbis[3-(meth)acryloyloxypropyl]silane, and methylphenylbis[3-(meth)acryloyloxypropyl]silane.

The polyfunctional (meth)acrylic monomer (A-2) is preferably an aromatic di(meth)acrylic acid ester represented by the following general formula (2). 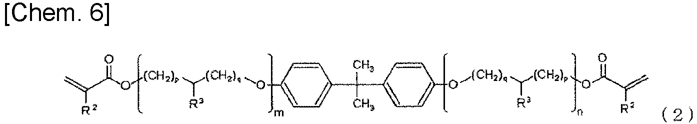 wherein R² represents a hydrogen atom or a methyl group, R³ represents a hydrogen atom or an alkyl group having 1 to 3 carbon atoms, p and q each independently represent an integer of 0 to 6, m and n each independently represent an integer of 0 or more, a plurality of R², R³, and p and q each may be the same or different from each other, and the average number of moles of alkyleneoxy group added as represented by an average of the sum of m and n per molecule is 2 to 10.

In the general formula (2), R² represents a hydrogen atom or a methyl group, R³ represents a hydrogen atom or an alkyl group having 1 to 3 carbon atoms, p and q each independently represent an integer of 0 to 6, and m and n each independently represent an integer of 0 or more. A plurality of R², R³, and p and q each may be the same or different from each other, Taking R³ as an example, a plurality of R³ may be the same, or may be different either partly or completely. The same is the case for R², p, and q. The average number of moles of alkyleneoxy group added as represented by an average of the sum of m and n per molecule is 2 to 10.

In the general formula (2), R² represents a hydrogen atom or a methyl group. In view of considerations such as past performance and safety as dental material, it is preferable that at least one of R² be a methyl group. More preferably, all of R² are methyl groups.

In the general formula (2), R³ represents a hydrogen atom or an alkyl group having 1 to 3 carbon atoms. Examples of the alkyl group having 1 to 3 carbon atoms include a methyl group, an ethyl group, and an isopropyl group. For advantages such as enhancement of the effectiveness of the present invention, R³ is preferably a hydrogen atom or a methyl group, more preferably a hydrogen atom.

In the general formula (2), p and q each independently represent an integer of 0 to 6. For advantages such as enhancement of the effectiveness of the present invention, the total of p and q in each unit represented by [-(CH₂)ₚ-CHR³-(CH₂)_{q}-O-] in general formula (2) is preferably 0 to 10, more preferably 1 to 7, even more preferably 1 to 3, particularly preferably 1 or 2, most preferably 1.

In the general formula (2), m and n each independently represent an integer of 0 or more. Preferably, m and n are each 0 to 10, more preferably 0 to 5, even more preferably 0 to 3.

In view of considerations such as ease of handling of the dental curable composition and the mechanical strength of the cured product, the average number of moles of alkyleneoxy group added (for example, the average number of moles of ethyleneoxy group added; described later) as represented by an average of the sum of m and n per molecule of an aromatic di(meth)acrylic acid ester represented by the general formula (2) is 2 to 10, preferably 2 to 5, more preferably 2 to 3, even more preferably 2.1 to 2.9.

Specific examples of aromatic di(meth)acrylic acid esters represented by the general formula (2) include 2,2-bis(4-(meth)acryloyloxy(poly)ethoxyphenyl)propane (the average number of moles of ethyleneoxy group added is 2 to 10). In view of considerations such as past performance and safety as dental material, 2,2-bis(4-methacryloyloxy(poly)ethoxyphenyl)propane (the average number of moles of ethyleneoxy group added is 2 to 10) is preferred. The 4-(meth)acryloyloxy(poly)ethoxyphenyl group in 2,2-bis(4-(meth)acryloyloxy(poly)ethoxyphenyl)propane may be a 4-(meth)acryloyloxyethoxyphenyl group or a 4-(meth)acryloyloxypolyethoxyphenyl group. One of the two 4-(meth)acryloyloxy(poly)ethoxyphenyl groups in 2,2-bis(4-methacryloyloxy(poly)ethoxyphenyl)propane may be a 4-(meth)acryloyloxyethoxyphenyl group, and the other may be a 4-(meth)acryloyloxypolyethoxyphenyl group. In view of ease of handling of the dental curable composition and the mechanical strength of the cured product, it is particularly preferable that the 2,2-bis(4-(meth)acryloyloxy(poly)ethoxyphenyl)propane comprise at least one selected from the group consisting of 2,2-bis(4-(meth)acryloyloxyethoxyphenyl)propane, 2-(4-(meth)acryloyloxydiethoxyphenyl)-2-(4-(meth)acryloyloxyethoxyphenyl)propane, and 2,2-bis(4-(meth)acryloyloxydiethoxyphenyl)propane.

The content of polyfunctional (meth)acrylic monomer (A-2) per 100 parts by mass of the polymerizable monomer (A) is 10 to 97 parts by mass, mere preferably 20 to 95 parts by mass, more preferably 30 to 90 parts by mass, particularly preferably 40 to 80 parts by mass, most preferably 50 to 70 parts by mass. By setting these upper limits, the polymerization shrinkage stress of the dental curable composition can be effectively reduced. By setting the foregoing lower limits, the dental curable composition causes less wear on opposing teeth, and can provide a high mechanical strength in cured form while having desirable water absorbency and good ease of handling. The polyfunctional (meth)acrylic monomer (A-2) may be used alone, or two or more thereof may be used in combination.

The polymerizable monomer (A) may consist of the polyfunctional (meth)acrylic monomer (A-1) and the polyfunctional (meth)acrylic monomer (A-2). However, the polymerizable monomer (A) may comprise another polymerizable monomer (A-3) (hereinafter, also referred to simply as "additional polymerizable monomer (A-3)"), in addition to the polyfunctional (meth)acrylic monomer (A-1) and the polyfunctional (meth)acrylic monomer (A-2). In view of considerations such as the polymerization shrinkage stress of the dental curable composition and the mechanical strength of the cured product, the total content of polyfunctional (meth)acrylic monomer (A-1) and polyfunctional (meth)acrylic monomer (A-2) in the polymerizable monomer (A) is preferably 20 mass% or more, more preferably 30 mass% or more, even more preferably 40 mass% or more, particularly preferably 50 mass% or more, and may be 90 mass% or 100 mass%. A certain preferred embodiment (W-1) may be a dental curable composition that comprises a polymerizable monomer (A), a filler (B), and a polymerization initiator (C), and in which the polymerizable monomer (A) contains the polyfunctional (meth)acrylic monomer (A-1) and the polyfunctional (meth)acrylic monomer (A-2), and the content of polyfunctional (meth)acrylic monomer (A-1) is 3 to 60 parts by mass, and the content of polyfunctional (meth)acrylic monomer (A-2) is 40 to 97 parts by mass in 100 parts by mass of the polymerizable monomer (A). In embodiment (W-1), the content of polyfunctional (meth)acrylic monomer (A-1) is preferably 5 to 60 parts by mass, and the content of polyfunctional (meth)acrylic monomer (A-2) is preferably 40 to 95 parts by mass. More preferably, the content of polyfunctional (meth)acrylic monomer (A-1) is 10 to 60 parts by mass, and the content of polyfunctional (meth)acrylic monomer (A-2) is 40 to 90 parts by mass. Even more preferably, the content of polyfunctional (meth)acrylic monomer (A-1) is 20 to 60 parts by mass, and the content of polyfunctional (meth)acrylic monomer (A-2) is 40 to 80 parts by mass.

The additional polymerizable monomer (A-3) may be a known polymerizable monomer used for curable compositions in dentistry. Particularly preferred for use are radical polymerizable monomers. Examples of the radical polymerizable monomers include esters of unsaturated carboxylic acids such as α-cyanoacrylic acid, (meth)acrylic acid, α-halogenated acrylic acid, crotonic acid, cinnamic acid, sorbic acid, maleic acid, and itaconic acid; (meth)acrylamide; derivatives of (meth)acrylamide; vinyl esters; vinyl ethers; mono-N-vinyl derivatives; and derivatives of styrene. The additional polymerizable monomer (A-3) may be used alone, or two or more thereof may be used in combination. Preferred are esters of unsaturated carboxylic acids, and derivatives of (meth)acrylamide. More preferred are esters of (meth)acrylic acid, and derivatives of (meth)acrylamide. Even more preferred are esters of (meth)acrylic acid. Other examples of the additional polymerizable monomer (A-3) include monofunctional (meth)acrylic monomers, esters of bifunctional (meth)acrylic acid, and esters of tri- and higher-functional (meth)acrylic acid. In a dental curable composition of the present invention, a polyfunctional (meth)acrylic monomer having both an aromatic ring and a hydroxyl group may be contained as an additional polymerizable monomer (A-3), provided that the constitution of the present invention is satisfied. However, in view of considerations such as ease of handling of the dental curable composition, it is preferable that such a polyfunctional (meth)acrylic monomer be not in excess. More preferably, the polymerizable monomer (A) is essentially free of such a polyfunctional (meth)acrylic monomer. The content of a polyfunctional (meth)acrylic monomer having both an aromatic ring and a hydroxyl group in polymerizable monomer (A) is preferably 10 mass% or less, more preferably 5 mass% or less, even more preferably 2 mass% or less, particularly preferably 0 mass%.

Examples of the (meth)acrylic monomers that can be used as additional polymerizable monomers (A-3) are as follows.

### (i) Monofunctional (Meth)Acrylic Acid Esters and Derivatives of (Meth)Acrylamide

Examples include methyl (meth)acrylate, isobutyl (meth)acrylate, benzyl (meth)acrylate, dodecyl (meth)acrylate, 2-(N,N-dimethylamino)ethyl (meth)acrylate, 2,3-dibromopropyl (meth)acrylate, 3-(meth)acryloyloxypropyltrimethoxysilane, 11-(meth)acryloyloxyundecyltrimethoxysilane, 2-hydroxyethyl (meth)acrylate, 3-hydroxypropyl (meth)acrylate, 4-hydroxybutyl (meth)acrylate, 6-hydroxyhexyl (meth)acrylate, 10-hydroxydecyl (meth)acrylate, propylene glycol mono(meth)acrylate, glycerin mono(meth)acrylate, erythritol mono(meth)acrylate, phenoxyethylene glycol (meth)acrylate, isobornyl (meth)acrylate, 3-phenoxybenzyl (meth)acrylate, N-methylol (meth)acrylamide, N-hydroxyethyl (meth)acrylamide, N, N-bis(hydroxyethyl)(meth)acrylamide, N,N-dimethyl (meth)acrylamide, N,N-diethyl (meth)acrylamide, N,N-di-n-propyl (meth)acrylamide, N-ethyl-N-methyl (meth)acrylamide, (meth)acryloylmorpholine, (meth)acryloyloxydodecylpyridinium bromide, (meth)acryloyloxydodecylpyridinium chloride, (meth)acryloyloxyhexadecylpyridinium bromide, and (meth)acryloyloxyhexadecylpyridinium chloride.

### (ii) Bifunctional (Meth)Acrylic Acid Esters

Examples include aromatic bifunctional (meth)acrylic acid esters (excluding those corresponding to the polyfunctional (meth)acrylic monomer (A-2)), and aliphatic bifunctional (meth)acrylic acid esters.

Examples of aromatic bifunctional (meth)acrylic acid esters include 2,2-bis[4-(3-(meth)acryloyloxy-2-hydroxypropoxy)phenyl]propane.

Examples of aliphatic bifunctional (meth)acrylic acid esters include glycerol di(meth)acrylate, ethylene glycol di(meth)acrylate, diethylene glycol di(meth)acrylate, triethylene glycol diacrylate, triethylene glycol dimethacrylate (hereinafter, also referred to as "3G"), propylene glycol di(meth)acrylate, butylene glycol di(meth)acrylate, neopentyl glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 1, 12-dodecanediol di(meth)acrylate, 1,2-bis(3-(meth)acryloyloxy-2-hydroxypropyloxy)ethane, tricyclodecanedimethanol di(meth)acrylate, 2,2,4-trimethylhexamethylenebis(2-carbamoyloxyethyl)di(meth)acrylate, and dicyclohexylbis[3-(meth)acryloyloxypropyl]silane.

### (iii) Tri- and Higher-Functional (Meth)AcrylicAcid Esters

Examples include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol tri(meth)acrylate, dipentaerythritol tetra(meth)acrylate, dipentaerythritol penta(meth)acrylate, N,N'-(2,2,4-trimethylhexamethylene)bis[2-(aminocarboxy)propane-1,3-diol]tetra(meth)acrylate, and 1,7- di(meth)acryloyloxy-2,2,6,6-tetra(meth)acryloyloxymethyl-4-oxaheptane.

In order for a dental curable composition to have desirable adhesive properties to adherends such as tooth structure, metal, and ceramic, there are cases where it may be preferable that the polymerizable monomer (A) additionally contain functional monomers with which the dental curable composition can have adhesive properties to such adherends.

Such functional monomers are monomers other than the polyfunctional (meth)acrylic monomer (A-1), polyfunctional (meth)acrylic monomer (A-2), and additional polymerizable monomer (A-3). In view of desirable adhesive properties to tooth structure and base metal, the functional monomers are, for example, acidic group-containing polymerizable monomers. Examples include polymerizable monomers having a phosphoric acid group, such as 2-(meth)acryloyloxyethyl dihydrogen phosphate, 10-(meth)acryloyloxydecyl dihydrogen phosphate, and 2-(meth)acryloyloxyethylphenyl hydrogen phosphate; and polymerizable monomers having a carboxylic acid group, such as 11-(meth)acryloyloxyundecane-1,1-dicarboxylic acid, and 4-(meth)acryloyloxyethoxycarbonylphthalic acid. In view of desirable adhesive properties to noble metals, the functional monomers are, for example, 10-mercaptodecyl (meth)acrylate, 6-(4-vinylbenzyl-n-propyl)amino-1,3,5-triazine-2,4-dithione, derivatives of thiouracil mentioned in JP 10(1998)-1473 A, or compounds having a sulfur element mentioned in JP 11(1999)-92461 A. In view of effective adhesion to ceramic, porcelain, and other curable compositions for dentistry, the functional monomers are, for example, silane coupling agents such as γ-(meth)acryloyloxypropyltrimethoxysilane. The functional monomer may be used alone, or two or more thereof may be used in combination.

The content of polymerizable monomer (A) in a dental curable composition of the present invention is not particularly limited. However, in view of properties such as ease of handling of the dental curable composition or the mechanical strength of the cured product, the content of polymerizable monomer (A) is preferably 1 mass% or more, more preferably 2 mass% or more, even more preferably 5 mass% or more, and may be 8 mass% or more, or 15 mass% or more, based on the mass of the dental curable composition. The content of polymerizable monomer (A) is 50 mass% or less, preferably 40 mass% or less, more preferably 30 mass% or less.

The polymerizable monomer (A) has a viscosity at 30°C of preferably 10 to 2,500 cP, more preferably 20 to 2,000 cP, even more preferably 30 to 1,500 cP, particularly preferably 40 to 1,000 cP, most preferably 50 to 500 cP. By setting these upper limits, the dental curable composition, with its relatively low viscosity, can have good ease of handling with the property to easily fill cavities. With the foregoing lower limits, the dental curable composition can have good ease of handling by reducing the runniness of the composition and making the paste more spreadable. The method of viscosity measurement is as described in the EXAMPLES section below.

### Filler (B)

A dental curable composition of the present invention comprises a filler (B). The filler (B) may be used alone, or two or more thereof may be used in combination.

The filler (B) has an average particle diameter that is preferably 0.01 µm or more, preferably 0.05 µm or more, more preferably 0.1 µm or more, even more preferably 0.2 µm or more, and is preferably 50 µm or less, more preferably 20 µm or less, even more preferably 10 µm or less, particularly preferably 7 µm or less. By setting these lower limits for the average particle diameter of filler (B), the dental curable composition can more effectively reduce stickiness, and ease of handling improves. By setting the foregoing upper limits for the average particle diameter of filler (B), the dental curable composition can more effectively reduce runniness or roughness, and ease of handling improves. When the filler (B) is an agglomerated particle (secondary particle), the average particle diameter of filler (B) means an average particle diameter of agglomerated particles (secondary particles).

The average particle diameter of filler (B) can be determined by a laser diffraction scattering method or electron microscopy. Specifically, a laser diffraction scattering method is convenient for the measurement of particles having a particle diameter of 0.1 µm or more, whereas electron microscopy is a convenient method of particle size measurement of ultrafine particles less than 0.1 µm. Here, 0.1 µm is a measured value by a laser diffraction scattering method. A laser diffraction scattering method may measure a particle size by volume, using, for example, a laser diffraction particle size distribution analyzer (e.g., SALD-2300 manufactured by Shimadzu Corporation) with ethanol or a 0.2% sodium hexametaphosphate aqueous solution used as dispersion medium. For electron microscopy, a scanning electron microscope (for example, SU3800, S-4000 manufactured by Hitachi High-Technologies Corporation) may be used. Specifically, for example, the average particle diameter can be determined by taking a electron micrograph of particles, and measuring the diameter of particles (at least 200 particles) observed in a unit field of the captured image, using image-analyzing particle-size-distribution measurement software (Mac-View manufactured by Mountech Co., Ltd.). Here, the particle diameter is determined as an arithmetic mean value of the maximum and minimum lengths of particles, and the average primary particle diameter is calculated from the number of particles and the particle diameter. The average particle diameter of filler (B) can be measured using the method specifically described in the EXAMPLES section below.

The overall particle shape of filler (B) is not particularly limited, and the filler (B) may be used in the form of an irregularly shaped powder or a spherical powder. An irregularly shaped filler (B) improves particularly the mechanical strength and wear resistance of the cured product of the dental curable composition. With a spherical filler (B), the dental curable composition can have improved ease of handling with smooth and readily spreadable paste properties. The overall shape of filler (B) can be appropriately selected according to the intended use of the dental curable composition.

The filler (B) in the present invention is broadly classified into inorganic filler and organic-inorganic composite filler.

Examples of inorganic fillers include various types of glasses. (For example, glasses containing silica as a main component, and, optionally, oxides of elements such as heavy metals, boron, and aluminum. Examples include glass powders of common compositions, for example, such as fused silica, quartz, soda-lime-silica glass, E glass, C glass, and borosilicate glass (PYREX^{®} glass); and dental glass powders, such as barium glass (GM27884 and 8235 manufactured by Schott, and E-2000 and E-3000 manufactured by Esstech), strontium-borosilicate glass (E-4000 manufactured by Esstech), lanthanum glass-ceramics (GM31684 manufactured by Schott), and fluoroaluminosilicate glass (GM35429, G018-091, and G018-117 manufactured by Schott)). Other examples of inorganic fillers include ceramics, composite oxides (e.g., silica-titania, silica-zirconia, silica-ytterbia), diatomaceous earth, kaolin, clay minerals (e.g., montmorillonite), activated earth, synthetic zeolite, mica, calcium fluoride, ytterbium fluoride, yttrium fluoride, calcium phosphate, barium sulfate, zirconium dioxide, titanium dioxide, and hydroxyapatite. The inorganic filler may be used alone, or two or more thereof may be used in combination. The inorganic filler is preferably one containing silica as a main component (containing at least 5 mass%, preferably at least 10 mass% silica).

In the present invention, the inorganic filler may have a form of an agglomerated particle formed by agglomeration of the inorganic filler. Commercially available inorganic fillers typically exist in the form of aggregates. The cohesion of commercially available inorganic fillers is so weak that these fillers break into particle sizes indicated by the manufacturer when 10 mg of its powder is added and ultrasonically dispersed at 40 W and 39 KHz for 30 minutes in 300 mL of water or in the same amount of a dispersion medium prepared by adding a surfactant (e.g., at most 5 mass% of sodium hexametaphosphate) to water. In contrast, the agglomerated particles in the present invention are strongly held together, and become hardly dispersed even under these conditions.

In a preferred method of preparing a strong agglomerate of particles from an aggregate of commercially available inorganic fillers, the inorganic filler is heated to a temperature range just below the temperature that melts the inorganic filler so that the adjoining inorganic filler particles under the applied heat lightly fuse together and increase cohesion. Here, the inorganic filler may have a form of an aggregate before heating, in order to control the shape of the agglomerated particle. An aggregate can be formed, for example, by applying pressure to the inorganic filler placed in a suitable container, or by dispersing the inorganic filler in a solvent, and removing the solvent using a method such as spray drying.

In another preferred method of preparing an agglomerated particle formed by strong aggregation of inorganic filler particles, a sol such as a silica sol, an alumina sol, a titania sol, or a zirconia sol prepared by using a wet method is dried using a method such as freeze drying or spray drying, and optionally subjected to a heat treatment. In this way, an agglomerated particle formed by strong aggregation of particles can be obtained with ease. Specific examples of the sols include fine spherical silica particles (Seahostar^{®} manufactured by Nippon Shokubai Co., Ltd. under this trade name; e.g., KE series, a surface-treated type), a silica organosol (OSCAL^{®} manufactured by JGC C & C under this trade name), a titania sol (QUEEN TITANIC series manufactured by Nissan Chemical Corporation under this trade name), a silica sol (SNOWTEX^{®} manufactured by Nissan Chemical Corporation under this trade name), an alumina sol (Aluminasol-100, Aluminasol-200, Aluminasol-520 manufactured by Nissan Chemical Corporation under these trade names), and a zirconia sol (NanoUse^{®} ZR series manufactured by Nissan Chemical Corporation under this trade name). The shape of the inorganic filler particle is not particularly limited, and may be appropriately selected for use.

The organic-inorganic composite filler means a filler containing the inorganic filler and a polymer of a polymerizable monomer.

The organic-inorganic composite filler in the present invention is preferably one in which inorganic filler particles having an average particle diameter of 5 µm or less are dispersed in an organic matrix. The average particle diameter is more preferably 2 µm or less, even more preferably 1 µm or less.

In the present invention, the method of production of the organic-inorganic composite filler is not particularly limited. For example, the organic-inorganic composite filler can be prepared by adding a known polymerizable monomer and a known polymerization initiator to the inorganic filler, polymerizing the filler mixture in paste form by a polymerization method such as solution polymerization, suspension polymerization, emulsion polymerization, or bulk polymerization, and pulverizing the resulting polymer.

The refractive index of filler (B) is not particularly limited. However, the transparency of the cured product can easily increase when the filler (B) has a refractive index close to the refractive indices of the components of the dental curable composition other than the filler (B). In this respect, the refractive index of filler (B) is preferably 1.45 or more, more preferably 1.50 or more, even more preferably 1.51 or more, and is preferably 1.63 or less, more preferably 1.60 or less, even more preferably 1.58 or less. The refractive index of filler (B) can be controlled by, for example, adjusting the types and proportions of metallic elements contained in the filler (B).

A surface treatment is not necessarily required for the filler (B). However, a surface treatment is preferred for a number of advantages, including improved compatibility with the polymerizable monomer (A) by making the surface of filler (B) hydrophobic, and increased amounts of filler (B). A surface treatment may be carried out using a surface treatment agent. The type of surface treatment agent is not particularly limited, and known surface treatment agents may be used. For example, the surface treatment agent may be a silane coupling agent, an organotitanium coupling agent, an organozirconium coupling agent, or an organoaluminum coupling agent. The surface treatment agent may be used alone, or two or more thereof may be used in combination. In view of availability and properties such as compatibility between polymerizable monomer (A) and filler (B), preferred are silane coupling agents.

The silane coupling agent is not particularly limited. However, the silane coupling agent is preferably a compound represented by the following general formula (3).

H₂C=CR⁶-CO-R⁷-(CH₂)_{z}-SiR⁸_{y}R⁹_{(3-y)} (3),

wherein R⁶ is a hydrogen atom or a methyl group, R⁷ is an oxygen atom, a sulfur atom, or -NR¹⁰-, where R¹⁰ is a hydrogen atom or an aliphatic group having 1 to 8 carbon atoms (may be linear, branched, or cyclic), R⁸ is a hydrolyzable group, R⁹ is a hydrocarbon group having 1 to 6 carbon atoms, y is an integer of 1 to 3, and z is an integer of 1 to 13, where a plurality of R⁸ and R⁹ each may be the same or different from each other.

In the general formula (3), R⁶ is a hydrogen atom or a methyl group, preferably a methyl group. R⁷ is an oxygen atom, a sulfur atom, or -NR¹⁰-, preferably an oxygen atom. R¹⁰ is a hydrogen atom or an aliphatic group having 1 to 8 carbon atoms (may be linear, branched, or cyclic), and the aliphatic group having 1 to 8 carbon atoms represented by R¹⁰ may be a saturated aliphatic group (e.g., an alkyl group, or a cycloalkylene group such as cyclohexyl) or an unsaturated aliphatic group (e.g., an alkenyl group, an alkynyl group). In view of availability and properties such as ease of production and chemical stability, the aliphatic group having 1 to 8 carbon atoms represented by R¹⁰ is preferably a saturated aliphatic group, more preferably an alkyl group. Examples of the alkyl group include methyl, ethyl, n-propyl, isopropyl, n-butyl, isobutyl, sec-butyl, tert-butyl, n-pentyl, isopentyl, n-hexyl, n-heptyl, 2-methylhexyl, and n-octyl. R¹⁰ is preferably a hydrogen atom or an alkyl group having 1 to 4 carbon atoms, more preferably a hydrogen atom or an alkyl group having 1 to 3 carbon atoms, even more preferably a hydrogen atom.

Examples of the hydrolyzable group represented by R⁸ in the general formula (3) include alkoxy groups such as methoxy, ethoxy, and butoxy; halogen atoms such as a chlorine atom and a bromine atom; and an isocyanate group. When a plurality of R⁸ exists, the plurality of R⁸ may be the same or different from each other. R⁸ is preferably an alkoxy group, more preferably methoxy or ethoxy, even more preferably methoxy.

Examples of the hydrocarbon group having 1 to 6 carbon atoms represented by R⁹ in the general formula (3) include an alkyl group having 1 to 6 carbon atoms (may be cyclic), an alkenyl group having 2 to 6 carbon atoms (may be cyclic), and an alkynyl group having 2 to 6 carbon atoms. When a plurality of R⁹ exists, the plurality of R⁹ may be the same or different from each other.

Examples of the alkyl group having 1 to 6 carbon atoms include methyl, ethyl, n-propyl, isopropyl, n-butyl, isobutyl, sec-butyl, tert-butyl, n-pentyl, isopentyl, neopentyl, tert-pentyl, n-hexyl, cyclopropyl, cyclobutyl, cyclopentyl, and cyclohexyl.

Examples of the alkenyl group having 2 to 6 carbon atoms include vinyl, allyl, 1-methylvinyl, 1-propenyl, butenyl, pentenyl, hexenyl, cyclopropenyl, cyclobutenyl, cyclopentenyl, and cyclohexenyl.

Examples of the alkynyl group having 2 to 6 carbon atoms include ethynyl, 1-propynyl, 2-propynyl, 1-butynyl, 1-methyl-2-propynyl, 2-butynyl, 3-butynyl, 1-pentynyl, 1-ethyl-2-propynyl, 2-pentynyl, 3-pentynyl, 1-methyl-2-butynyl, 4-pentynyl, 1-methyl-3-butynyl, 2-methyl-3-butynyl, 1-hexynyl, 2-hexynyl, 1-ethyl-2-butynyl, 3-hexynyl, 1-methyl-2-pentynyl, 1-methyl-3-pentynyl, 4-methyl-1-pentynyl, 3-methyl-1-pentynyl, 5-hexynyl, and 1-ethyl-3-butynyl.

In the general formula (3), y is an integer of 1 to 3, preferably 2 or 3, more preferably 3. In the general formula (3), z is an integer of 1 to 13, preferably an integer of 2 to 12, more preferably an integer of 3 to 11.

Specific examples of the silane coupling agent represented by the general formula (3) include methacryloyloxymethyltrimethoxysilane, 2-methacryloyloxyethyltrimethoxysilane, 3-methacryloyloxypropyltrimethoxysilane, 4-methacryloyloxybutyltrimethoxysilane, 5-methacryloyloxypentyltrimethoxysilane, 6-methacryloyloxyhexyltrimethoxysilane, 7-methacryloyloxyheptyltrimethoxysilane, 8-methacryloyloxyoctyltrimethoxysilane, 9-methacryloyloxynonyltrimethoxysilane, 10-methacryloyloxydecyltrimethoxysilane, 11-methacryloyloxyundecyltrimethoxysilane, 12-methacryloyloxydodecyltrimethoxysilane, 13-methacryloyloxytridecyltrimethoxysilane, 11-methacryloyloxyundecyldichloromethylsilane, 11-methacryloyloxyundecyltrichlorosilane, and 12-methacryloyloxydodecyldimethoxymethylsilane. The silane coupling agent may be used alone, or two or more thereof may be used in combination. In view of availability, 3-methacryloyloxypropyltrimethoxysilane is preferred. In view of improved compatibility between polymerizable monomer (A) and filler (B), 8-methacryloyloxyoctyltrimethoxysilane, 9-methacryloyloxynonyltrimethoxysilane, 10-methacryloyloxydecyltrimethoxysilane, and 11-methacryloyloxyundecyltrimethoxysilane are preferred.

The surface treatment method is not particularly limited, and a known method may be used.

The amount of surface treatment agent used is not particularly limited, and may be, for example, at least 1 part by mass relative to 100 parts by mass of the filler before surface treatment. The amount of surface treatment agent is preferably at least 5 parts by mass, more preferably at least 6 parts by mass, even more preferably at least 8 parts by mass, particularly preferably at least 10 parts by mass, most preferably at least 20 parts by mass, and is preferably at most 50 parts by mass, more preferably at most 45 parts by mass, even more preferably at most 40 parts by mass. By setting these lower limits for the amount of surface treatment agent, the mechanical strength of the cured product can more easily improve. By setting the foregoing upper limits for the amount of surface treatment agent, a decrease of the mechanical strength of the cured product due to the excessive surface treatment agent can be reduced.

The content of filler (B) in a dental curable composition of the present invention (the content of filler (B) after surface treatment when subjected to a surface treatment) is 50 mass% or more, particularly preferably 65 mass% or more based on the mass of the dental curable composition, and is preferably 97 mass% or less, more preferably 95 mass% or less, even more preferably 90 mass% or less based on the mass of the dental curable composition. By setting these lower limits for the content of filler (B), the cured product can have improved mechanical strength, and the dental curable composition can have improved ease of handling because of effectively reduced stickiness. By setting the foregoing upper limits for the content of filler (B), the dental curable composition can be prevented from becoming too hard, and ease of handling improves.

A dental curable composition of the present invention may contain an organic filler. Examples of the organic filler include polymethylmethacrylate, polyethylmethacrylate, a methyl methacrylate-ethyl methacrylate copolymer, crosslinked polymethylmethacrylate, crosslinked polyethylmethacrylate, polyamides, polyvinyl chloride, polystyrene, chloroprene rubber, nitrile rubber, an ethylene-vinyl acetate copolymer, a styrenebutadiene copolymer, an acrylonitrile-styrene copolymer, and an acrylonitrile-styrenebutadiene copolymer. These may be used alone, or two or more thereof may be used as a mixture. The shape of the organic filler is not particularly limited, and the particle diameter of the filler may be appropriately selected for use. In view of considerations such as the ease of handling and mechanical strength of the composition obtained, the average particle diameter of the organic filler is preferably 0.001 to 50 µm, more preferably 0.001 to 10 µm.

### Polymerization Initiator (C)

A dental curable composition of the present invention comprises a polymerization initiator (C). The polymerization initiator (C) may be selected from polymerization initiators commonly used in industry, preferably those used in dentistry. Particularly preferred for use are photopolymerization initiators and chemical polymerization initiators. The polymerization initiator (C) may be used alone, or two or more thereof may be used in appropriate combinations.

Examples of the photopolymerization initiators include (bis)acylphosphine oxides, ketals, α-diketones, and coumarins.

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, benzoyldi(2,6-dimethylphenyl)phosphonate, and salts thereof (for example, sodium salts, potassium salts, and ammonium salts). Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, bis(2,5,6-trimethylbenzoyl)-2,4,4-trimethylpentylphosphine oxide, and salts thereof (for example, sodium salts, potassium salts, and ammonium salts).

Particularly preferred among these (bis)acylphosphine oxides are 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and sodium salts of 2,4,6-trimethylbenzoylphenylphosphine oxide.

Examples of the ketals include benzyl dimethyl ketal, and benzyl diethyl ketal.

Examples of the α-diketones include diacetyl, benzyl, camphorquinone, 2,3-pentadione, 2,3-octadione, 9,10-phenanthrenequinone, 4,4'-oxybenzyl, and acenaphthenequinone. Particularly preferred is camphorquinone for its maximum absorption wavelength occurring in the visible light region.

Examples of the coumarin compounds include compounds mentioned in JP 9(1997)-3109 A and JP 10(1998)-245525 A, such as 3,3'-carbonylbis(7-diethylaminocoumarin), 3-(4-methoxybenzoyl)coumarin, 3-thienoyl coumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoyl coumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazol-2-ylidene)acetyl]coumarin, 3-[(1-methylnaphtho[1,2-d]thiazol-2-ylidene)acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazolyl)-7-(diethylamino)coumarin, 3-(2-benzothiazolyl)-7-(dibutylamino)coumarin, 3-(2-benzoimidazolyl)-7-(diethylamino)coumarin, 3-(2-benzothiazolyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-[3-[4-(dimethylamino)phenyl]-1-oxo-2-propenyl]-2,3,6,7-tetrahydro-1,1,7,7-tetramethyl-1H,5H,11H-[1]benzopyrrano[6,7,8-ij]quinolizin-11-one, and 10-(2-benzothiazolyl)-2,3,6,7-tetrahydro-1,1,7,7-tetramethyl-1H,5H,11H-[1]benzopyrrano[6,7,8-ij]quinolizin-11-one.

Particularly preferred among these coumarin compounds are 3,3'-carbonylbis(7-diethylaminocoumarin), and 3,3'-carbonylbis(7-dibutylaminocoumarin).

When the photopolymerization initiator is at least one selected from the group consisting of a (bis)acylphosphine oxide, an α-diketone, and a coumarin compound, a dental curable composition can be provided that has desirable photocurability both in the visible light region and the near ultraviolet region, and that shows sufficient photocurability regardless of whether the light source used is a halogen lamp, a light emitting diode (LED), or a xenon lamp.

The chemical polymerization initiators are preferably organic peroxides. The organic peroxides used as chemical polymerization initiators are not particularly limited, and known organic peroxides may be used. Typical examples of such organic peroxides include ketone peroxides, hydroperoxides, diacyl peroxides, dialkyl peroxides, peroxyketals, peroxyesters, and peroxydicarbonates.

Examples of the ketone peroxides include methyl ethyl ketone peroxide, methyl isobutyl ketone peroxide, methylcyclohexanone peroxide, and cyclohexanone peroxide.

Examples of the hydroperoxides include 2,5-dimethylhexane-2,5-dihydroperoxide, diisopropylbenzenehydroperoxide, cumenehydroperoxide, t-butylhydroperoxide, and 1,1,3,3-tetramethylbutylhydroperoxide.

Examples of the diacyl peroxides include acetyl peroxide, isobutyryl peroxide, benzoyl peroxide, decanoyl peroxide, 3,5,5-trimethylhexanoyl peroxide, 2,4-dichlorobenzoyl peroxide, and lauroyl peroxide.

Examples of the dialkyl peroxides include di-t-butyl peroxide, dicumyl peroxide, t-butylcumyl peroxide, 2,5-dimethyl-2,5-di(t-butylperoxy)hexane, 1,3-bis(t-butylperoxyisopropyl)benzene, and 2,5-dimethyl-2,5-di(t-butylperoxy)-3-hexyne.

Examples of the peroxyketals include 1,1-bis(t-butylperoxy)-3,3,5-trimethylcyclohexane, 1,1-bis(t-butylperoxy)cyclohexane, 2,2-bis(t-butylperoxy)butane, 2,2-bis(t-butylperoxy)octane, and n-butyl 4,4-bis(t-butylperoxy)valerate.

Examples of the peroxyesters include α-cumyl peroxyneodecanoate, t-butyl peroxyneodecanoate, t-butyl peroxypivalate, 2,2,4-trimethylpentyl peroxy-2-ethylhexanoate, t-amyl peroxy-2-ethylhexanoate, t-butyl peroxy-2-ethylhexanoate, di-t-butyl peroxyisophthalate, di-t-butyl peroxyhexahydroterephthalate, t-butyl peroxy-3,3,5-trimethylhexanoate, t-butyl peroxyacetate, t-butyl peroxybenzoate, and t-butyl peroxymaleic acid.

Examples of the peroxydicarbonates include di-3-methoxyperoxydicarbonate, di(2-ethylhexyl)peroxydicarbonate, bis(4-t-butylcyclohexyl)peroxydicarbonate, diisopropylperoxydicarbonate, di-n-propylperoxydicarbonate, di(2-ethoxyethyl)peroxydicarbonate, and diallylperoxydicarbonate.

From an overall balance of safety, storage stability, and radical generating potential, preferred among these organic peroxides are diacyl peroxides, more preferably benzoyl peroxide.

The content of polymerization initiator (C) in a dental curable composition of the present invention is not particularly limited. However, in view of considerations such as the curability of the dental curable composition obtained, the content of polymerization initiator (C) is preferably 0.001 mass% or more, more preferably 0.01 mass% or more, even more preferably 0.02 mass% or more, particularly preferably 0.1 mass% or more based on the total mass of the dental curable composition. For considerations such as a possibility of the polymerization initiator (C) precipitating out of the dental curable composition when the content is too high, the content of polymerization initiator (C) based on the total mass of the dental curable composition is preferably 30 mass% or less, more preferably 20 mass% or less, even more preferably 15 mass% or less, particularly preferably 10 mass% or less, and may be 5 mass% or less, 2 mass% or less, or 1 mass% or less.

### Polymerization Accelerator (D)

A dental curable composition of the present invention may further comprise a polymerization accelerator (D). Examples of the polymerization accelerator (D) include amines, sulfinic acids and salts thereof, derivatives of barbituric acid, borate compounds, triazine compounds, copper compounds, tin compounds, vanadium compounds, halogen compounds, aldehydes, thiol compounds, sulfites, bisulfites, and thiourea compounds. The polymerization accelerator (D) may be used alone, or two or more thereof may be used in combination. A certain preferred embodiment may be a dental curable composition in which the polymerization initiator (C) comprises a photopolymerization initiator, and the dental curable composition additionally comprises a polymerization accelerator (D) that is a tertiary amine. The tertiary amine is preferably a tertiary aromatic amine.

The amines can be classified into aliphatic amines and aromatic amines. Examples of the aliphatic amines include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. In view of curability and storage stability of the dental curable composition, preferred for use are tertiary aliphatic amines, more preferably N-methyldiethanolamine and triethanolamine.

Examples of the aromatic amines include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, ethyl 4-(N,N-dimethylamino)benzoate, methyl 4-(N,N-dimethylamino)benzoate, 2-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. In view of the ability to impart desirable curability to the dental curable composition, preferred for use is at least one selected from the group consisting of N,N-bis(2-hydroxyethyl)-p-toluidine, ethyl 4-(N,N-dimethylamino)benzoate, 2-butoxyethyl 4-(N,N-dimethylamino)benzoate, and 4-(N,N-dimethylamino)benzophenone.

Examples of the sulfinic acids and salts thereof include p-toluenesulfinic acid, sodium p-toluenesulfinate, potassium p-toluenesulfinate, lithium p-toluenesulfinate, calcium p-toluenesulfinate, benzenesulfinic acid, sodium benzenesulfinate, potassium benzenesulfinate, lithium benzenesulfinate, calcium benzenesulfinate, 2,4,6-trimethylbenzenesulfinic acid, sodium 2,4,6-trimethylbenzenesulfinate, potassium 2,4,6-trimethylbenzenesulfinate, lithium 2,4,6-trimethylbenzenesulfinate, calcium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, lithium 2,4,6-triethylbenzenesulfinate, calcium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, lithium 2,4,6-triisopropylbenzenesulfinate, and calcium 2,4,6-triisopropylbenzenesulfinate. Particularly preferred are sodium benzenesulfinate, sodium p-toluenesulfinate, and sodium 2,4,6-triisopropylbenzenesulfinate.

Examples of the derivatives of barbituric acid include barbituric acid, 1,3-dimethylbarbituric acid, 1,3-diphenylbarbituric acid, 1,5-dimethylbarbituric acid, 5-butylbarbituric acid, 5-ethylbarbituric acid, 5-isopropylbarbituric acid, 5-cyclohexylbarbituric acid, 1,3,5-trimethylbarbituric acid, 1,3-dimethyl-5-ethylbarbituric acid, 1,3-dimethyl-5-n-butylbarbituric acid, 1,3-dimethyl-5-isobutylbarbituric acid, 1,3-dimethyl-5-cyclopentylbarbituric acid, 1,3-dimethyl-5-cyclohexylbarbituric acid, 1,3-dimethyl-5-phenylbarbituric acid, 1-cyclohexyl-1-ethylbarbituric acid, 1-benzyl-5-phenylbarbituric acid, 5-methylbarbituric acid, 5-propylbarbituric acid, 1,5-diethylbarbituric acid, 1-ethyl-5-methylbarbituric acid, 1-ethyl-5-isobutylbarbituric acid, 1,3-diethyl-5-butylbarbituric acid, 1-cyclohexyl-5-methylbarbituric acid, 1-cyclohexyl-5-ethylbarbituric acid, 1-cyclohexyl-5-octylbarbituric acid, 1-cyclohexyl-5-hexylbarbituric acid, 5-butyl-1-cyclohexylbarbituric acid, 1-benzyl-5-phenylbarbituric acid, thiobarbituric acid, and salts of these (particularly preferred are alkali metal salts or alkali earth metal salts). Examples of the salts of barbituric acid include sodium 5-butylbarbiturate, sodium 1,3,5-trimethylbarbiturate, and sodium 1-cyclohexyl-5-ethylbarbiturate.

Particularly preferred as derivatives of barbituric acid are 5-butylbarbituric acid, 1,3,5-trimethylbarbituric acid, 1-cyclohexyl-5-ethylbarbituric acid, 1-benzyl-5-phenylbarbituric acid, and sodium salts of these barbituric acids.

The borate compounds are preferably arylborate compounds. Examples of the arylborate compounds include borate compounds having 1 to 4 aryl groups per molecule. In view of storage stability, preferred as arylborate compounds are borate compounds having 3 or 4 aryl groups per molecule. Examples of the borate compounds having 3 or 4 aryl groups per molecule include monoalkyl triphenylboron, monoalkyl tri(p-chlorophenyl)boron, monoalkyl tri(p-fluorophenyl)boron, monoalkyl tri[3,5-bis(trifluoromethyl)phenyl]boron, monoalkyl tri[3,5-bis(1,1,1,3,3,3-hexafluoro-2-methoxy-2-propyl)phenyl]boron, monoalkyl tri(p-nitrophenyl)boron, monoalkyl tri(m-nitrophenyl)boron, monoalkyl tri(p-butylphenyl)boron, monoalkyl tri(m-butylphenyl)boron, monoalkyl tri(p-butyloxyphenyl)boron, monoalkyl tri(m-butyloxyphenyl)boron, monoalkyl tri(p-octyloxyphenyl)boron, monoalkyl tri(m-octyloxyphenyl)boron (the alkyl groups in these examples are, for example, n-butyl, n-octyl, or n-dodecyl), and salts of these (sodium salts, lithium salts, potassium salts, magnesium salts, tetrabutylammonium salts, tetramethylammonium salts, tetraethylammonium salts, methylpyridinium salts, ethylpyridinium salts, butylpyridinium salts, methylquinolinium salts, ethylquinolinium salts, and butylquinolinium salts).

Examples of the borate compounds having 4 aryl groups per molecule include tetraphenyl boron, tetrakis(p-chlorophenyl)boron, tetrakis(p-fluorophenyl)boron, tetrakis[3,5-bis(trifluoromethyl)phenyl]boron, tetrakis[3,5-bis(1,1,1,3,3,3-hexafluoro-2-methoxy-2-propyl)phenyl]boron, tetrakis(p-nitrophenyl)boron, tetrakis(m-nitrophenyl)boron, tetrakis(p-butylphenyl)boron, tetrakis(m-butylphenyl)boron, tetrakis(p-butyloxyphenyl)boron, tetrakis(m-butyloxyphenyl)boron, tetrakis(p-octyloxyphenyl)boron, tetrakis(m-octyloxyphenyl)boron, (p-fluorophenyl)triphenylboron, [3,5-bis(trifluoromethyl)phenyl]triphenylboron, (p-nitrophenyl)triphenylboron, (m-butyloxyphenyl)triphenylboron, (p-butyloxyphenyl)triphenylboron, (m-octyloxyphenyl)triphenylboron, (p-octyloxyphenyl)triphenylboron, and salts of these (sodium salts, lithium salts, potassium salts, magnesium salts, tetrabutylammonium salts, tetramethylammonium salts, tetraethylammonium salts, methylpyridinium salts, ethylpyridinium salts, butylpyridinium salts, methylquinolinium salts, ethylquinolinium salts, and butylquinolinium salts).

Examples of the triazine compounds include 2,4,6-tris(trichloromethyl)-s-triazine, 2,4,6-tris(tribromomethyl)-s-triazine, 2-methyl-4,6-bis(trichloromethyl)-s-triazine, 2-methyl-4,6-bis(tribromomethyl)-s-triazine, 2-phenyl-4,6-bis(trichloromethyl)-s-triazine, 2-(p-methoxyphenyl)-4,6-bis(trichloromethyl)-s-triazine, 2-(p-methylthiophenyl)-4,6-bis(trichloromethyl)-s-triazine, 2-(p-chlorophenyl)-4,6-bis(trichloromethyl)-s-triazine, 2-(2,4-dichlorophenyl)-4,6-bis(trichloromethyl)-s-triazine, 2-(p-bromophenyl)-4,6-bis(trichloromethyl)-s-triazine, 2-(p-tolyl)-4,6-bis(trichloromethyl)-s-triazine, 2-n-propyl-4,6-bis(trichloromethyl)-s-triazine, 2-(α,α,β-trichloroethyl)-4,6-bis(trichloromethyl)-s-triazine, 2-styryl-4,6-bis(trichloromethyl)-s-triazine, 2-[2-(p-methoxyphenyl)ethenyl]-4,6-bis(trichloromethyl)-s-triazine, 2-[2-(o-methoxyphenyl)ethenyl]-4,6-bis(trichloromethyl)-s-triazine, 2-[2-(p-butoxyphenyl)ethenyl]-4,6-bis(trichloromethyl)-s-triazine, 2-[2-(3,4-dimethoxyphenyl)ethenyl]-4,6-bis(trichloromethyl)-s-triazine, 2-[2-(3,4,5-trimethoxyphenyl)ethenyl]-4,6-bis(trichloromethyl)-s-triazine, 2-(1-naphthyl)-4,6-bis(trichloromethyl)-s-triazine, 2-(4-biphenylyl)-4,6-bis(trichloromethyl)-s-triazine, 2-[2-{N,N-bis(2-hydroxyethyl)amino}ethoxy]-4,6-bis(trichloromethyl)-s-triazine, 2-[2-{N-hydroxyethyl-N-ethylamino}ethoxy]-4,6-bis(trichloromethyl)-s-triazine, 2-[2-{N-hydroxyethyl-N-methylamino}ethoxy]-4,6-bis(trichloromethyl)-s-triazine, and 2-[2-{N,N-diallylamino}ethoxy]-4,6-bis(trichloromethyl)-s-triazine.

Among these triazine compounds, particularly preferred in view of polymerization activity is 2,4,6-tris(trichloromethyl)-s-triazine. Particularly preferred in view of storage stability are 2-phenyl-4,6-bis(trichloromethyl)-s-triazine, 2-(p-chlorophenyl)-4,6-bis(trichloromethyl)-s-triazine, and 2-(4-biphenylyl)-4,6-bis(trichloromethyl)-s-triazine. The triazine compounds may be used alone, or two or more thereof may be used in combination.

Preferred for use as copper compounds are, for example, copper acetylacetonate, copper(II) acetate, copper oleate, copper(II) chloride, and copper(II) bromide.

Examples of the tin compounds include di-n-butyltin dimaleate, di-n-octyltin dimaleate, di-n-octyltin dilaurate, and di-n-butyltin dilaurate. Particularly preferred are din-octyltin dilaurate, and di-n-butyltin dilaurate.

The vanadium compounds are preferably vanadium compounds with valences of IV and/or V. Examples of vanadium compounds with valences of IV and/or V include compounds mentioned in JP 2003-96122 A, for example, such as vanadium(IV) oxide, vanadium(IV)oxy acetylacetonate, vanadyl oxalate, vanadyl sulfate, vanadium(IV) oxobis(1-phenyl-1,3-butanedionate), bis(maltolato)oxovanadium(IV), vanadium(V) oxide, sodium metavanadate, and ammonium metavanadate.

Preferred for use as halogen compounds are, for example, dilauryldimethylammonium chloride, lauryldimethylbenzylammonium chloride, benzyltrimethylammonium chloride, tetramethylammonium chloride, benzyldimethylcetylammonium chloride, and dilauryldimethylammonium bromide.

Examples of the aldehydes include terephthalaldehyde, and derivatives of benzaldehyde. Examples of the derivatives of benzaldehyde include dimethylaminobenzaldehyde, p-methoxybenzaldehyde, p-ethoxybenzaldehyde, and p-n-octyloxybenzaldehyde. In view of curability, preferred for use is p-n-octyloxybenzaldehyde.

Examples of the thiol compounds include 3-mercaptopropyltrimethoxysilane, 2-mercaptobenzooxazole, decanethiol, and thiobenzoic acid.

Examples of the sulfites include sodium sulfite, potassium sulfite, calcium sulfite, and ammonium sulfite.

Examples of the bisulfites include sodium bisulfite and potassium bisulfite.

Examples of the thiourea compounds include 1-(2-pyridyl)-2-thiourea, thiourea, methylthiourea, ethylthiourea, N,N'-dimethylthiourea, N,N'-diethylthiourea, N,N'-di-n-propylthiourea, N,N'-dicyclohexylthiourea, trimethylthiourea, triethylthiourea, tri-n-propylthiourea, tricyclohexylthiourea, tetramethylthiourea, tetraethylthiourea, tetra-n-propylthiourea, and tetracyclohexylthiourea.

When a dental curable composition of the present invention contains the polymerization accelerator (D), the content of polymerization accelerator (D) is not particularly limited. However, in view of considerations such as the curability of the dental curable composition obtained, the content of polymerization accelerator (D) based on the total mass of the dental curable composition is preferably 0.001 mass% or more, more preferably 0.01 mass% or more, even more preferably 0.02 mass% or more, and may be 0.03 mass% or more, 0.05 mass% or more, or 0.1 mass% or more. For considerations such as a possibility of the polymerization accelerator (D) precipitating out of the dental curable composition when the content is too high, the content of polymerization accelerator (D) based on the total mass of the dental curable composition is preferably 30 mass% or less, more preferably 20 mass% or less, even more preferably 10 mass% or less, particularly preferably 5 mass% or less, and may be 2 mass% or less, 1 mass% or less, or 0.5 mass% or less.

### Additive (E)

A dental curable composition of the present invention may optionally comprise an additive (E), for example, such as a polymerization inhibitor, a ultraviolet absorber, an antioxidant, an antimicrobial agent, a dispersant, a pH adjuster, a fluorescent agent, or a colorant (a pigment, a dye), other than the polymerizable monomer (A), filler (B), polymerization initiator (C), and polymerization accelerator (D) described above. The additive (E) may be used alone, or two or more thereof may be used in combination.

Examples of the polymerization inhibitor include 3,5-di-t-butyl-4-hydroxytoluene, hydroquinone, dibutyl hydroquinone, dibutyl hydroquinone monomethyl ether, hydroquinone monomethyl ether, and 2,6-di-t-butylphenol. These may be used alone, or two or more thereof may be used in combination. Examples of the ultraviolet absorber include benzotriazole compounds such as 2-(2-hydroxyphenyl)benzotriazole, 2-(2-hydroxy-5-methylphenyl)benzotriazole, 2-(2-hydroxy-5-ethylphenyl)benzotriazole, 2-(2-hydroxy-5-propylphenyl)benzotriazole, 2-(2-hydroxy-5-tert-octylphenyl)benzotriazole, and 2-(2-hydroxy-3-tert-butyl-5-methylphenyl)-5-chloro-2H-benzotriazole (Tinuvin 326); and benzoimidazole compounds.
These may be used alone, or two or more thereof may be used in combination.

### Method of Production of Dental curable composition

A method of preparation of a dental curable composition of the present invention is not particularly limited, and a dental curable composition of the present invention can be obtained by combining the components in predetermined amounts. The components may be combined in any order, at once or in two or more separate portions. Optionally, the components may be mixed or kneaded, or may be subjected to degassing, for example, vacuum degassing. The resultant dental curable composition may be charged into a single container (e.g., a syringe) to prepare a one-pack (one-paste) type dental curable composition.

### Uses

A dental curable composition of the present invention is not limited to particular uses, and may be used as a variety of dental materials. Specifically, a dental curable composition of the present invention can be suitably used as, for example, a dental composite resin (for example, a composite resin for filling cavities, a composite resin for abutment construction, a composite resin for dental caps), a denture base resin, a denture base liner, an impression material, a luting material (for example, a resin cement, a resin-added glass ionomer cement), a dental bonding agent (for example, an orthodontics adhesive, an adhesive for application to cavities), a tooth fissure sealant, a resin block for CAD/CAM, a temporary crown, or an artificial teeth material. Because of desirable properties such as good ease of handling and the property to provide desirable mechanical strength and polishability for the cured product while having a low polymerization shrinkage stress with a consistency to easily fill cavities, a dental curable composition of the present invention is particularly suited as a dental composite resin.

The present invention encompasses, within the scope of the claims, embodiments combining the foregoing features, provided that such combinations made in various forms within the technical idea of the present invention can produce the effects of the present invention.

### EXAMPLES

The following describes the present invention in greater detail by way of Examples and Comparative Examples. It is to be noted, however, that the present invention is not limited to the following Examples. The following summarizes details of Examples, including the test methods and materials used in Examples.

### Test Methods

### Average Particle Diameter of Filler

The fillers below were measured for average particle diameter by volume with a laser diffraction particle size distribution analyzer (SALD-2300, manufactured by Shimadzu Corporation), using ethanol and a 0.2% sodium hexametaphosphate aqueous solution as dispersion media.

### Viscosity of Polymerizable Monomer

Viscosity was measured for 100 parts by mass in total of the polymerizable monomers (A) shown in Table 1. The measurement was made for a 0.6 mL sample at 30°C with a 0.8° × R24 conical rotor, using a viscometer (a TV-30E type viscometer manufactured by Toki Sangyo Co., Ltd.; JIS K-7117-2:1999 compliant; a cone-plate type). The measurement was started after 1 minute of preheating, and the measured value after 5 minutes was determined as the viscosity.

### Flexural Strength and Flexural Modulus of Cured Product

The curable compositions for dentistry obtained in Examples and Comparative Examples were degassed in vacuum, and each was charged into a stainless-steel mold (dimensions: 2 mm × 2 mm × 25 mm). With the dental composition being pressed between glass slides from top and bottom, light was applied through the glass slides from both sides to cure the dental composition and obtain a cured product specimen. Here, light was applied at 5 points each side, 10 seconds at each point, using a dental LED photoirradiator for polymerization (PenCure 2000 manufactured by J. Morita Corp. under this trade name). A total of five cured products were prepared for each Example and Comparative Example. The cured product was stored in 37°C distilled water for 24 hours after being taken out of the mold. For the measurement of flexural strength and flexural modulus, the specimens were tested in a three-point flexural test conducted according to JIS T 6514:2015 and ISO 4049:2009 at a span length of 20 mm and a crosshead speed of 1 mm/min, using a precision universal testing machine (Autograph AG-I, 100 kN, manufactured by Shimadzu Corporation under this trade name) (N = 5). From the measured values, a mean value was calculated for each specimen to find the flexural strength and flexural modulus. The flexural strength is preferably 80 MPa or more, more preferably 90 MPa or more, even more preferably 100 MPa or more, particularly preferably 120 MPa or more. The upper limits of flexural strength are not particularly limited, and may be, for example, 200 MPa or less, or 150 MPa or less. The flexural modulus is preferably 8 GPa or less, more preferably 2 to 7.5 GPa, even more preferably 4 to 7 GPa because polymerization shrinkage stress and wear on opposing teeth can be reduced with the flexural modulus falling in these ranges.

### Polymerization Shrinkage Stress

The dental curable composition obtained in each Example and Comparative Example was charged into a ring-shaped mold (stainless steel; 5.5 mm in inner diameter × 0.8 mm in thickness) placed on a 4.0 mm-thick glass plate. The glass plate was used after being sandblasted with an alumina powder having a particle diameter of 50 µm. A stainless-steel jig (Ø = 5 mm), coupled to a universal testing machine (Autograph AG-I, 100 kN, manufactured by Shimadzu Corporation under this trade name), was then placed on the dental curable composition filling the mold. The dental curable composition was cured by applying light for 20 seconds through the glass plate, using a dental LED photoirradiator for polymerization (PenCure 2000 manufactured by J. Morita Corp. under this trade name). The polymerization shrinkage stress due to curing of the dental curable composition by a polymerization reaction initiated by photoirradiation was measured with the universal testing machine (N = 5). Table 1 shows the mean value calculated from values from each specimen. Smaller values of polymerization shrinkage stress are preferred because it means that a contraction gap is less likely to occur. Smaller values of polymerization shrinkage stress are also preferred from a procedure standpoint because a larger amount of dental curable composition can be filled at once when the polymerization shrinkage stress is smaller. The polymerization shrinkage stress is preferably 12.0 MPa or less, more preferably 10.0 MPa or less, even more preferably 9.0 MPa or less.

### Consistency of Composition

A glass plate was placed on measured 0.25 cc of a paste of each dental curable composition obtained in Examples and Comparative Examples, and the paste was crushed under a 1 kg load applied for 30 seconds via the glass plate. The resulting paste spread in a circular disc shape was then measured for maximum diameter and minimum diameter, and the mean value (mm) of these diameters was determined as the consistency (N = 3). Table 1 shows the mean value calculated from values from each specimen. Larger consistencies indicate that the paste is softer. In view of ease of handling of the paste, the consistency is preferably 11.0 mm or more, more preferably 13.0 mm or more, even more preferably 14.0 mm or more, particularly preferably 15.0 mm or more. The consistency is preferably 100 mm or less, more preferably 80 mm or less.

### Water Absorption

Water absorption of each dental curable composition obtained in Examples and Comparative Examples was measured according to JIS T 6514:2015 and ISO4049:2009 (N = 5). Table 1 shows the mean value calculated from values from each specimen. The dental curable composition was cured using a dental LED photoirradiator for polymerization (PenCure2000 manufactured by J. Morita Corp. under this trade name). The water absorption is preferably 35 µg/ mm³ or less, more preferably 25 µg/ mm³ or less, even more preferably 20 µg/ mm³ or less.

### Wear Volume of Opposing Teeth

### Leinfelder Abrasion Test

Preparation of Bovine Enamel Surface: A surface of a bovine tooth was polished in succession with wet abrasive sheets (#80, #1000, #3000; manufactured by Nihon Kenshi Co., Ltd.) to fabricate a specimen having a smooth, mirror-finished enamel surface. The specimen was embedded in a metal mold with a fast polymerizable resin (UNIFAST II, manufactured by GC under this trade name), without burying the enamel surface (at least 5 mm was exposed in diameter).

Preparation of Dental curable composition: The dental curable composition obtained in each Example and Comparative Example was filled into a hemispherical plastic mold (Ø = 11 mm). With the mold set up on a cylindrical plastic bar, the dental curable composition was irradiated with light for 3 minutes, using an LED polymerizer (α Light V, manufactured by J. Morita Corp. under this trade name). After photoirradiation, the surface of the resultant cured product was polished in succession with wet abrasive sheets (#1000, #2000, #3000; manufactured by Nihon Kenshi Co., Ltd.).

The bovine enamel surface and the cured product of the dental curable composition were set on a Leinfelder abrasion tester (manufactured by NAVIC). With the bovine tooth immersed in distilled water, the cured product was pressed against the bovine enamel surface under a 7.9 kg test load at room temperature with a 30° rotation applied under the maximum load before releasing the applied load and rotating the cured product back to the original position (0°). This procedure was repeated 400,000 times. After the test, the volume of enamel surface lost by abrasion was measured as the wear volume of opposing tooth, using a laser microscope (VR-3200 manufactured by Keyence under this trade name) (N = 6). Table 1 shows the mean value calculated from values from each specimen. The wear volume of opposing tooth is preferably 0.3 mm³ or less, more preferably 0.25 mm³ or less, even more preferably 0.2 mm³ or less.

### Materials

### Polyfunctional (meth)acrylic monomer (A-1)

9BG: Polybutylene glycol dimethacrylate (In the formula (4) below, the average number of moles of butylene glycol group added is 9; C/O = 4; a total of 45 carbon and oxygen atoms constituting the backbone.)

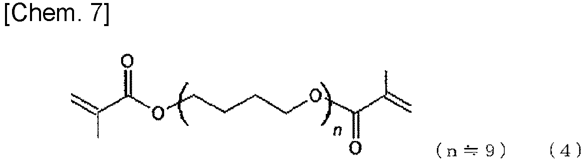

### Polyfunctional (meth)acrylic monomer (A-2)

D2.6E: 2,2-Bis(4-methacryloyloxypolyethoxyphenyl)propane (average number of moles of ethyleneoxy group added is 2.6)
D7E: 2,2-Bis(4-methacryloyloxypolyethoxyphenyl)propane (average number of moles of ethyleneoxy group added is 7)

### Additional polymerizable monomer (A-3)

3G: Triethylene glycol dimethacrylate (C/O = 2; a total of 9 carbon and oxygen atoms constituting the backbone)
9G: Polyethylene glycol dimethacrylate (average number of moles of ethylene glycol group added is 9; C/O = 2; a total of 27 carbon and oxygen atoms constituting the backbone)
14G: Polyethylene glycol dimethacrylate (average number of moles of ethylene glycol group added is 14; (C/O = 2; a total of 42 carbon and oxygen atoms constituting the backbone)
3PG: Tripropylene glycol dimethacrylate (In the formula (5) below, C/O = 3, a total of 9 carbon and oxygen atoms constituting the backbone.) 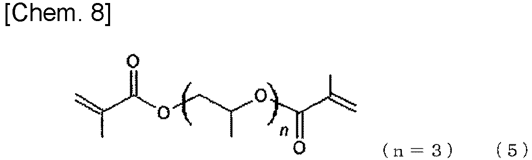
7PG: Polypropylene glycol dimethacrylate (In the formula (6) below, average number of moles of propylene glycol group added is 7; C/O = 3; a total of 21 carbon and oxygen atoms constituting the backbone.) 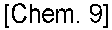 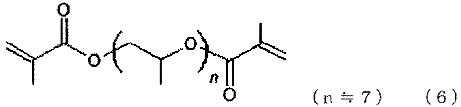
Bis-GMA: 2,2-Bis[4-(3-methacryloyloxy-2-hydroxypropoxy)phenyl]propane

### Filler (B)

Fillers obtained in the following Production Examples were used.

### Production Example 1

### Production of NF180

A three-neck flask was charged with 100 parts by mass of a commercially available barium glass (GM27884 NanoFine 180, manufactured by Schott), 7 parts by mass of 3-methacryloyloxypropyltrimethoxysilane, and 173 parts by mass of toluene, and the mixture was stirred at room temperature for 2 hours. After removing toluene under reduced pressure, the mixture was vacuum dried at 40°C for 16 hours, and was heated at 90°C for 3 hours to obtain an inorganic filler (NF180) having a surface-treated layer. The inorganic filler (NF180) produced had an average particle diameter of 0.2 µm.

### Production Example 2

### Production of UF2.0

A three-neck flask was charged with 100 parts by mass of a commercially available barium glass (UltraFine UF2.0, manufactured by Schott), 5 parts by mass of 3-methacryloyloxypropyltrimethoxysilane, and 173 parts by mass of toluene, and the mixture was stirred at room temperature for 2 hours. After removing toluene under reduced pressure, the mixture was vacuum dried at 40°C for 16 hours, and was heated at 90°C for 3 hours to obtain an inorganic filler (UF2.0) having a surface-treated layer. The inorganic filler (UF2.0) produced had an average particle diameter of 2.0 µm.

### Production Example 3

### Production of NF-CF

A paste was prepared by adding and mixing 100 parts by mass of NF180 (the inorganic filler produced above) with 100 parts by mass of a 1:1 polymerizable monomer mixture of Bis-GMA and 3G (mass ratio) dissolving 1 mass% of azobisisobutyronitrile (AIBN) as a polymerization initiator. The paste was subjected to thermal polymerization at 100°C for 5 hours in a reduced pressure atmosphere. The resultant cured product was pulverized with a vibration ball mill until the particles had an average particle diameter of 5 µm. This was followed by a surface treatment, in which 100 g of the powder produced was refluxed at 90°C for 5 hours in a 200 mL ethanol solution of 2 mass% γ-methacryloyloxypropyltrimethoxysilane to obtain an organic-inorganic composite filler (NF-CF) having an average particle diameter of 5 µm.

### Polymerization initiator (C)

CQ: Camphorquinone

### Polymerization accelerator (D)

PDE: Ethyl 4-(N,N-dimethylamino)benzoate

### Additive (E)

TN326: Tinuvin 326 (a ultraviolet absorber manufactured by BASF Japan)
BHT: 3,5-Di-t-butyl-4-hydroxytoluene (polymerization inhibitor)

### Examples 1 to 5 and Comparative Examples 1 to 7

These materials were mixed at ordinary temperature (23°C) in the dark in the combinations and proportions shown in Table 1 to prepare curable compositions for dentistry. The curable compositions for dentistry were tested using the methods described above. The results are presented in Table 1.

**[Table 1]**

| Components (parts by mass) | | Examples | | | | | Comparative Examples | | | | | | |
|---|---|---|---|---|---|---|---|---|---|---|---|---|---|
| | | 1 | 2 | 3 | 4 | 5 | 1 | 2 | 3 | 4 | 5 | 6 | 7 |
| Polyfunctional (meth)acrylic monomer (A-1) | 9BG | 10 | 40 | 55 | 40 | 20 | 70 | | | | | | 40 |
| Polyfunctional (meth)acrylic monomer (A-2) | D2.6E | 90 | 60 | 45 | 60 | | 30 | 60 | 60 | 60 | 60 | 60 | |
| | D7E | | | | | 80 | | | | | | | |
| Additional polymerizable monomer (A-3) | 3G | | | | | | | 40 | | | | | |
| | 9G | | | | | | | | 40 | | | | |
| | 14G | | | | | | | | | 40 | | | |
| | 3PG | | | | | | | | | | 40 | | |
| | 7PG | | | | | | | | | | | 40 | |
| | Bis-GMA | | | | | | | | | | | | 60 |
| Total | | 100 | 100 | 100 | 100 | 100 | 100 | 100 | 100 | 100 | 100 | 100 | 100 |
| Viscosity of polymerizable monomer (A) (cP) | | 420 | 196 | 150 | 196 | 222 | 127 | 50 | 112 | 204 | 67 | 117 | 2673 |
| Polymerizable monomer (A) | | 27 | 27 | 27 | 23 | 27 | 27 | 27 | 27 | 27 | 27 | 27 | 27 |
| Filler (B) | NF180 | 3 | 3 | 3 | 7 | 3 | 3 | 3 | 3 | 3 | 3 | 3 | 3 |
| | UF2.0 | 70 | 70 | 70 | 21 | 70 | 70 | 70 | 70 | 70 | 70 | 70 | 70 |
| | NF-CF | | | | 49 | | | | | | | | |
| Polymerization initiator (C) | CQ | 0.4 | 0.4 | 0.4 | 0.4 | 0.4 | 0.4 | 0.4 | 0.4 | 0.4 | 0.4 | 0.4 | 0.4 |
| Polymerization accelerator (D) | PDE | 0.4 | 0.4 | 0.4 | 0.4 | 0.4 | 0.4 | 0.4 | 0.4 | 0.4 | 0.4 | 0.4 | 0.4 |
| Additive (E) | TN326 | 0.5 | 0.5 | 0.5 | 0.5 | 0.5 | 0.5 | 0.5 | 0.5 | 0.5 | 0.5 | 0.5 | 0.5 |
| | BHT | 0.1 | 0.1 | 0.1 | 0.1 | 0.1 | 0.1 | 0.1 | 0.1 | 0.1 | 0.1 | 0.1 | 0.1 |
| Total | | 101.4 | 101.4 | 101.4 | 101.4 | 101.4 | 101.4 | 101.4 | 101.4 | 101.4 | 101.4 | 101.4 | 101.4 |
| Flexural strength | MPa | 136 | 111 | 110 | 106 | 129 | 67 | 148 | 141 | 78 | 129 | 138 | 120 |
| Flexural modulus | GPa | 7.7 | 5.0 | 4.7 | 4.4 | 6.1 | 2.8 | 9.2 | 8.4 | 7.2 | 8.4 | 8.4 | 8.2 |
| Polymerization shrinkage stress | MPa | 10.4 | 9.3 | 9.0 | 8.3 | 10.7 | 6.9 | 14.5 | 13.2 | 10.2 | 13.3 | 12.1 | 11.2 |
| Consistency | mm | 14.6 | 19.5 | 18.3 | 18.0 | 18.5 | 20.1 | 24.6 | 18.2 | 16.8 | 25.7 | 23.0 | 12.5 |
| Water absorption | µg/mm³ | 5.6 | 5.5 | 8.6 | 13.1 | 24.1 | 19.0 | 7.9 | 38.1 | 49.2 | 10.2 | 26.3 | 17.2 |
| Wear volume of opposing tooth | mm³ | 0.27 | 0.21 | 0.18 | 0.18 | 0.26 | 0.14 | 0.34 | 0.36 | 0.18 | 0.30 | 0.39 | 0.35 |

As can be seen in Table 1, the curable compositions for dentistry of the present invention have high flexural strength and low water absorption, despite the low polymerization shrinkage stress. The curable compositions for dentistry of the present invention also have low flexural modulus, and the wear volume of opposing tooth is small. Another property is that the paste does not have an overly low consistency, making it easier to fill cavities.

### INDUSTRIAL APPLICABILITY

A dental curable composition of the present invention has a small polymerization shrinkage stress and a consistency to easily fill cavities, and, in the form of a cured product, has excellent mechanical strength with low water absorbency while causing reduced wear on opposing teeth. This makes the dental curable composition suited for applications such as dental composite resins.

## Claims

1. A dental curable composition comprising a polymerizable monomer (A), a filler (B), and a polymerization initiator (C),
the polymerizable monomer (A) containing a polyfunctional (meth)acrylic monomer (A-1) having no aromatic ring but having a moiety (X) constituted by an alkoxylene repeating unit having 1 to 15 carbon atoms, and a polyfunctional (meth)acrylic monomer (A-2) having an aromatic ring but no hydroxyl group,
the moiety (X) having a C/O of 2.5 to 10 as a ratio of a total number of carbon atoms and a total number of oxygen atoms in the moiety (X),
the moiety (X) having a backbone with a total of 25 to 60 constituent carbon atoms and oxygen atoms altogether,
the polyfunctional (meth)acrylic monomer (A-1) being 3 to 60 parts by mass in 100 parts by mass of the polymerizable monomer (A),
the polyfunctional (meth)acrylic monomer (A-2) is 10 to 97 parts by mass in 100 parts by mass of the polymerizable monomer (A),
the filler (B) is selected from inorganic filler and organic-inorganic composite filler,
the content of the filler (B) is 50 mass% or more, based on the mass of the dental curable composition.

2. The dental curable composition according to claim 1, wherein the polymerizable monomer (A) has a viscosity at 30°C of 0.01 to 2.5 Pa·s(10 to 2,500 cP).

3. The dental curable composition according to any of claim 1 or claim 2, wherein the polyfunctional (meth)acrylic monomer (A-1) is a di(meth)acrylic acid ester represented by the following general formula (1), 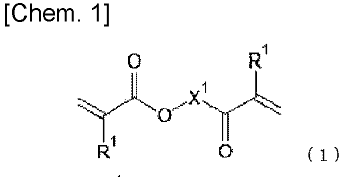
wherein R¹ represents a hydrogen atom or a methyl group,
X¹ is an alkoxylene repeating unit having 1 to 15 carbon atoms, in which C/O as a ratio of a total number of constituent carbon atoms and a total number of constituent oxygen atoms is 2.5 to 10, and X¹ has a backbone with a total of 25 to 60 constituent carbon atoms and oxygen atoms altogether, and
a plurality of R¹ may be the same or different from each other.

4. The dental curable composition according to claim 3, wherein the alkoxylene repeating unit constituting X¹ has 3 to 5 carbon atoms.

5. The dental curable composition according to any one of claims 1 to 4, wherein the polyfunctional (meth)acrylic monomer (A-1) is a polybutylene glycol dimethacrylate represented by the following general formula (4) (average number of moles of butylene glycol group added: 5 to 11),

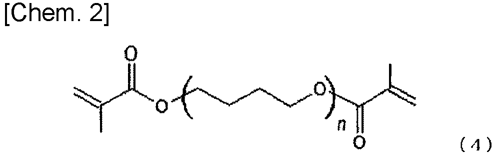

6. The dental curable composition according to any one of claims 1 to 5, wherein the polyfunctional (meth)acrylic monomer (A-2) is an aromatic di(meth)acrylic acid ester represented by the following general formula (2), 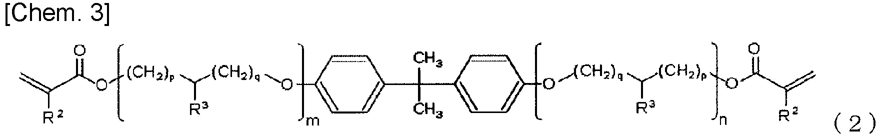 wherein R² represents a hydrogen atom or a methyl group, R³ represents a hydrogen atom or an alkyl group having 1 to 3 carbon atoms, p and q each independently represent an integer of 0 to 6, m and n each independently represent an integer of 0 or more, a plurality of R², R³, and p and q each may be the same or different from each other, and the average number of moles of alkyleneoxy group added as represented by an average of the sum of m and n per molecule is 2 to 10.

7. The dental curable composition according to any one of claims 1 to 6, wherein the filler (B) has an average particle diameter of 0.01 to 50 µm, measured by laser diffraction as described in the description.

8. The dental curable composition according to any one of claims 1 to 7, wherein a cured product of the curable composition has a flexural modulus of 8 GPa or less.

9. The dental curable composition according to any one of claims 1 to 8, wherein the curable composition has a polymerization shrinkage stress of 12.0 MPa or less.

## Patentansprüche

1. Zahnmedizinische, aushärtbare Zusammensetzung, umfassend ein polymerisierbares Monomer (A), einen Füllstoff (B) und einen Polymerisationsinitiator (C),
wobei das polymerisierbare Monomer (A) ein polyfunktionelles (Meth)acryl-Monomer (A-1) ohne aromatischen Ring, aber mit einem aus einer Alkoxylen-Wiederholungseinheit mit 1 bis 15 Kohlenstoffatomen bestehenden Molekülteil (X) enthält, und ein polyfunktionelles (Meth)acryl-Monomer (A-2) mit einem aromatischen Ring, aber ohne Hydroxylgruppe, enthält,
wobei der Molekülteil (X) ein C/O-Verhältnis von 2,5 zu 10 als ein Verhältnis der Gesamtzahl von Kohlenstoffatomen zu der Gesamtzahl von Sauerstoffatomen im Molekülteil (X) aufweist,
wobei der Molekülteil (X) ein Rückgrat aus insgesamt 25 bis 60 Kohlenstoff- und Sauerstoffatomen aufweist,
wobei das polyfunktionelle (Meth)acryl-Monomer (A-1) 3 bis 60 Massenanteile von 100 Massenanteilen des polymerisierbaren Monomers (A) ausmacht,
wobei das polyfunktionelle (Meth)acryl-Monomer (A-2) 10 bis 97 Massenanteile von 100 Massenanteilen des polymerisierbaren Monomers (A) ausmacht,
wobei der Füllstoff (B) aus anorganischen Füllstoffen und organisch-anorganischen Verbundfüllstoffen ausgewählt ist,
wobei der Anteil des Füllstoffs (B) mindestens 50 Masse-% ausmacht, bezogen auf die Masse der zahnmedizinischen, aushärtbaren Zusammensetzung.

2. Zahnmedizinische, aushärtbare Zusammensetzung nach Anspruch 1, wobei das polymerisierbare Monomer (A) bei 30 °C eine Viskosität von 0,01 bis 2,5 Pa·s (10 bis 2.500 cP) aufweist.

3. Zahnmedizinische, aushärtbare Zusammensetzung nach einem der Ansprüche 1 oder 2, wobei es sich bei dem polyfunktionellen (Meth)acryl-Monomer (A-1) um einen Di(meth)acrylsäureester der folgenden allgemeinen Formel (1) handelt, 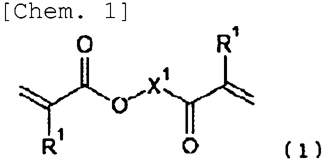
wobei R¹ ein Wasserstoffatom oder eine Methylgruppe darstellt,
wobei X¹ eine Alkoxylen-Wiederholungseinheit mit 1 bis 15 Kohlenstoffatomen ist, wobei C/O als ein Verhältnis von der Gesamtzahl von Kohlenstoffatomen zu der Gesamtzahl von Sauerstoffatomen 2,5 zu 10 beträgt und X¹ ein Rückgrat mit insgesamt 25 bis 60 Kohlenstoff- und Sauerstoffatomen aufweist, und eine Vielzahl von R¹ gleich oder voneinander verschieden sein kann.

4. Zahnmedizinische, aushärtbare Zusammensetzung nach Anspruch 3, wobei die Alkoxylen-Wiederholungseinheit X¹ 3 bis 5 Kohlenstoffatome aufweist.

5. Zahnmedizinische, aushärtbare Zusammensetzung nach einem der Ansprüche 1 bis 4, wobei es sich bei dem polyfunktionellen (Meth)acryl-Monomer (A-1) um ein Polybutylenglykoldimethacrylat der folgenden allgemeinen Formel (4) handelt (durchschnittliche Molzahl der zugesetzten Butylenglykolgruppen: 5 bis 11),

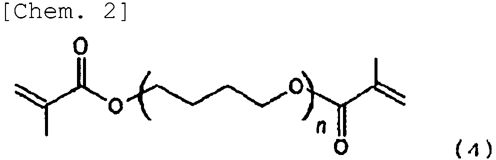

6. Zahnmedizinische, aushärtbare Zusammensetzung nach einem der Ansprüche 1 bis 5, wobei es sich bei dem polyfunktionellen (Meth)acryl-Monomer (A-2) um einen aromatischen Di(meth)acrylsäureester der folgenden allgemeinen Formel (2) handelt, 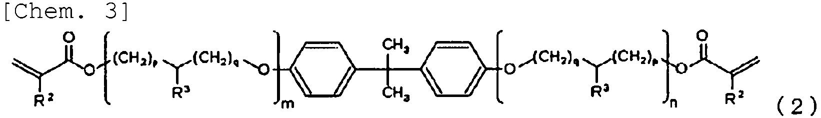 wobei R² ein Wasserstoffatom oder eine Methylgruppe darstellt, R³ ein Wasserstoffatom oder eine Alkylgruppe mit 1 bis 3 Kohlenstoffatomen darstellt, p und q jeweils unabhängig voneinander eine ganze Zahl von 0 bis 6 darstellen, m und n jeweils unabhängig voneinander eine ganze Zahl von 0 oder mehr darstellen, eine Vielzahl von R², R³, p und q jeweils gleich oder voneinander verschieden sein können und die durchschnittliche Molzahl der zugesetzten Alkylenoxygruppe, dargestellt durch den Durchschnitt der Summe von m und n pro Molekül, 2 bis 10 beträgt.

7. Zahnmedizinische, aushärtbare Zusammensetzung nach einem der Ansprüche 1 bis 6, wobei der Füllstoff (B) einen mittleren Partikeldurchmesser von 0,01 bis 50 µm aufweist, gemessen mittels Laserbeugung, wie in der Beschreibung beschrieben.

8. Zahnmedizinische, aushärtbare Zusammensetzung nach einem der Ansprüche 1 bis 7, wobei ein ausgehärtetes Erzeugnis der aushärtbaren Zusammensetzung einen Biegemodul von höchstens 8 GPa aufweist.

9. Zahnmedizinische, aushärtbare Zusammensetzung nach einem der Ansprüche 1 bis 8, wobei die ausgehärtete Zusammensetzung eine Polymerisationsschrumpfungsspannung von höchstens 12,0 MPa aufweist.

## Revendications

1. Composition dentaire durcissable comprenant un monomère polymérisable (A), une charge (B) et un initiateur de polymérisation (C),
le monomère polymérisable (A) contenant un monomère (méth)acrylique polyfonctionnel (A-1) n'ayant pas de cycle aromatique mais ayant un groupement (X) constitué par un motif répétitif alcoxylène possédant 1 à 15 atomes de carbone, et un monomère (méth)acrylique polyfonctionnel (A-2) ayant un cycle aromatique mais pas de groupe hydroxyle,
le groupement (X) ayant un C/O de 2,5 à 10 en tant que rapport d'un nombre total d'atomes de carbone et d'un nombre total d'atomes d'oxygène dans le groupement (X),
le groupement (X) ayant un squelette avec un total de 25 à 60 atomes de carbone et atomes d'oxygène constitutifs au total,
le monomère (méth)acrylique polyfonctionnel (A-1) étant de 3 à 60 parties en masse dans 100 parties en masse du monomère polymérisable (A),
le monomère (méth)acrylique polyfonctionnel (A-2) est 10 à 97 parties en masse dans 100 parties en masse du monomère polymérisable (A),
la charge (B) est choisie parmi une charge inorganique et une charge composite organique-inorganique,
la teneur de la charge (B) est de 50 % en masse ou plus, sur la base de la masse de la composition dentaire durcissable.

2. Composition dentaire durcissable selon la revendication 1, dans laquelle le monomère polymérisable (A) a une viscosité à 30 °C de 0,01 à 2,5 Pa s (10 à 2 500 cP).

3. Composition dentaire durcissable selon l'une quelconque parmi la revendication 1 ou la revendication 2, dans laquelle le monomère (méth)acrylique polyfonctionnel (A-1) est un diester d'acide (méth)acrylique représenté par la formule générale suivante (1), 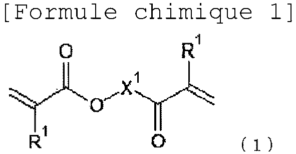
dans laquelle R¹ représente un atome d'hydrogène ou un groupe méthyle,
X¹ est un motif répétitif alcoxylène ayant 1 à 15 atomes de carbone, où C/O en tant que rapport d'un nombre total d'atomes de carbone constitutifs et d'un nombre total d'atomes d'oxygène constitutifs est de 2,5 à 10, et X¹ a un squelette ayant un total de 25 à 60 atomes de carbone et atomes d'oxygène constitutifs au total, et
une pluralité de R¹ peuvent être identiques ou différents les uns des autres.

4. Composition dentaire durcissable selon la revendication 3, dans laquelle le motif répétitif alcoxylène constitutif X¹ a 3 à 5 atomes de carbone.

5. Composition dentaire durcissable selon l'une quelconque des revendications 1 à 4, dans laquelle le monomère (méth)acrylique polyfonctionnel (A-1) est un diméthacrylate de polybutylène glycol représenté par la formule générale suivante (4) (nombre moyen de moles de groupe butylène glycol ajouté : 5 à 11),

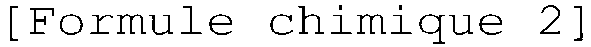

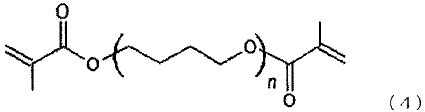

6. Composition dentaire durcissable selon l'une quelconque des revendications 1 à 5, dans laquelle le monomère (méth)acrylique polyfonctionnel (A-2) est un diester d'acide (méth)acrylique aromatique représenté par la formule générale suivante (2), 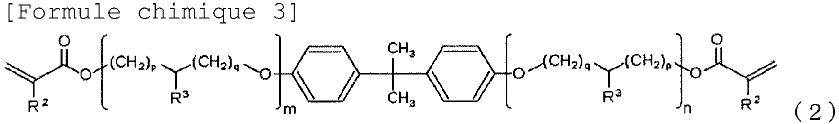 dans laquelle R² représente un atome d'hydrogène ou un groupe méthyle, R³ représente un atome d'hydrogène ou un groupe alkyle ayant 1 à 3 atomes de carbone, p et q représentent chacun indépendamment un entier de 0 à 6, m et n représentent chacun indépendamment un entier de 0 ou plus, une pluralité de R², R³, et p et q peuvent chacun être identiques ou différents les uns des autres, et le nombre moyen de moles de groupe alkylènoxy ajouté comme représenté par une moyenne de la somme de m et n par molécule est 2 à 10.

7. Composition dentaire durcissable selon l'une quelconque des revendications 1 à 6, dans laquelle la charge (B) a un diamètre moyen de particule de 0,01 à 50 µm, mesuré par diffraction laser comme décrit dans la description.

8. Composition dentaire durcissable selon l'une quelconque des revendications 1 à 7, dans laquelle un produit durci de la composition durcissable a un module de flexion de 8 GPa ou moins.

9. Composition dentaire durcissable selon l'une quelconque des revendications 1 à 8, dans laquelle la composition durcissable a une contrainte de retrait de polymérisation de 12,0 MPa ou moins.
